# EUROPÄISCHE PATENTSCHRIFT

(11) **EP 0 617 923 B1**
(45) Veröffentlichungstag und Bekanntmachung des Hinweises auf die Patenterteilung: **13.11.1996**
(21) Anmeldenummer: 94103421.7
(22) Anmeldetag: 07.03.1994
(51) Int. Cl.: A61B 17/04

(54) **Chirurgische Nähmaschine**
Surgical sewing machine
Machine chirurgicale à coudre

(30) Priorität: 31.03.1993 DE 4310555
(43) Veröffentlichungstag der Anmeldung: 05.10.1994
(73) Patentinhaber: J. STROBEL & SÖHNE GmbH & CO, D-80339 München (DE)
(72) Erfinder: Klundt, Kurt, D-67732 Hirschhorn (DE); Moll, Philipp, D-52078 Aachen (DE); Schlöndorff, Georg, Prof. Dr. med., D-52159 Roetgen (DE)
(74) Vertreter: Oedekoven, Wolf-Dieter, Dipl.-Ing.

(56) Entgegenhaltungen:
- WO-A-85/05025
- GB-A- 2 050 448

## Beschreibung

Die Erfindung bezieht sich auf eine chirurgische Nähmaschine der im Oberbegriff des Patentanspruchs 1 angegebenen Art.

Solche chirurgischen Nähmaschinen sind bekannt (DE- C 37 02 015). Sie weisen zwei kreisrunde und jeweils drehbar gelagerte Scheiben zum Zusammenklemmen der miteinander zu vernähenden Geweberänder auf, wobei eine Scheibe seitlich bewegbar und auf die andere Scheibe zu federbelastet ist und in Vorschubrichtung schrittweise antreibbar sein kann. Die Geweberandabstützvorrichtung besteht aus zwei stationären Stützfingern, welche sich auf der der Nadel und dem Schlingenfänger zugewandten Seite der beiden Klemmscheiben jeweils entlang des Umfangs der einen bzw. der anderen Klemmscheibe in Vorschubrichtung auf die Bewegungsbahn der Nadel zu erstrecken, um die miteinander zu vernähenden Geweberänder seitlich zu halten, wenn sie sich in dem von den Stützfingern gebildeten Trichter in Vorschubrichtung auf die Nadelbewegungsbahn zu bewegen.

Der Erfindung liegt die Aufgabe zugrunde, eine chirurgische Nähmaschine der im Oberbegriff des Patentanspruchs 1 angegebenen Gattung zu schaffen, bei welcher die Abstützung der miteinander zu vernähenden Geweberänder verbessert ist und in Verbindung damit auch der Vorschub der miteinander zu vernähenden Geweberänder verbessert werden kann.

Diese Aufgabe ist durch die im kennzeichnenden Teil des Patentanspruchs 1 angegebenen Merkmale gelöst. Vorteilhafte Ausgestaltungen der erfindungsgemäßen chirurgischen Nähmaschine sind in den restlichen Patentansprüchen angegeben.

Nachstehend sind mehrere Ausführungsformen der erfindungsgemäßen chirurgischen Nähmaschine anhand von Zeichnungen beispielsweise beschrieben. Darin zeigt:
- Fig. 1: perspektivisch eine längsgeschnittene erste Ausführungsform;
- Fig. 2: die Vorderansicht in Richtung des Pfeils II in Fig. 1 in anderem Maßstab;
- Fig. 3: den Längsschnitt entlang der Linie III-III in Fig. 2 in anderem Maßstab;
- Fig. 4: die Draufsicht in Richtung des Pfeils IV in Fig. 3 bei abgenommenem Deckel;
- Fig. 5: den Querschnitt entlang der Linie V-V in Fig. 3 im Maßstab von Fig. 2;
- Fig. 6: den Querschnitt entlang der Linie VI-VI in Fig. 3 im Maßstab von Fig. 2;
- Fig. 7: die Seitenansicht in Richtung des Pfeils VII in Fig. 6;
- Fig. 8: die Draufsicht in Richtung des Pfeils VIII in Fig. 6;
- Fig. 9: den Längsschnitt gemäß Fig. 3 einer zweiten Ausführungsform in anderem Maßstab;
- Fig. 10: die Draufsicht in Richtung des Pfeils X in Fig. 9;
- Fig. 11a: den Querschnitt entlang der Linie XI-XI in Fig. 9;
- Fig. 11b: den Querschnitt gemäß Fig. 11a, wobei die beiden Pinzettenschenkel sich jedoch in der Öffnungsstellung befinden;
- Fig. 12: den Querschnitt entlang der Linie XII-XII in Fig. 9;
- Fig. 13: den Längsschnitt entlang der Linie XIII-XIII in Fig. 11a;
- Fig. 14: perspektivisch die beiden in Fig. 11a, 11b und 12 rechten Betätigungshebel des einen Pinzettenschenkels;
- Fig. 15: perspektivisch die beiden in Fig. 11a, 11b und 12 linken Betätigungshebel des anderen Pinzettenschenkels;
- Fig. 16: perspektivisch eine längsgeschnittene dritte Ausführungsform;
- Fig. 17: die Vorderansicht in Richtung des Pfeils XVII in Fig. 16 in anderem Maßstab;
- Fig. 18: den Längsschnit entlang der Linie XVIII-XVIII in Fig. 17 im Maßstab von Fig. 16;
- Fig. 19: den Längsschnitt entlang der Linie XIX-XIX in Fig. 18;
- Fig. 20: den Querschnitt entlang der Linie XX-XX in Fig. 19 im Maßstab von Fig. 17;
- Fig. 21: den Querschnitt entlang der Linie XXI-XXI in Fig. 19 im Maßstab von Fig. 17;
- Fig. 22: den Querschnitt entlang der Linie XXII-XXII in Fig. 19 im Maßstab von Fig. 17;
- Fig. 23: den Querschnitt entlang der Linie XXIII-XXIII in Fig. 19 im Maßstab von Fig. 17;
- Fig. 24: den Querschnitt entlang der Linie XXIV-XXIV in Fig. 19 im Maßstab von Fig. 17.

Die dargestellten chirurgischen Nähmaschinen weisen jeweils eine Bogennadel 1, einen gabelförmigen Schwinggreifer 2 und einen elektrischen Gleichstrommotor 3 zum Antrieb der Bogennadel 1 und des Schwinggreifers 2 auf. Die Bogennadel 1 ist an einem Nadelhebel 4 vorgesehen, welcher radial von einer Nadelwelle 5 absteht. Der Schwinggreifer 2 ist an einer Greiferstange 6 angeordnet.

Der Gleichstrommotor 3 treibt über ein Untersetzungsgetriebe 7 eine Steuerwalze 8 mit einem Nadelantriebsexzenter 9, einem Greiferantriebsexzenter 10 und einer Greiferantriebsnut 11 an. Das Untersetzungsgetriebe 7 besteht aus einem auf der Ausgangswelle 12 des Gleichstrommotors 3 vorgesehenen Ritzel 13 und einem zur Steuerwalze 8 koaxialen und damit verbundenen Zahnrad 14, mit welchem das Ritzel 13 kämmt.

Der Nadelantriebsexzenter 9 wirkt mit der Nadelwelle 5 über eine nicht dargestellte Pleuelstange zusammen, welche an einem ebenfalls nicht dargestellten Betätigungsarm angreift, der radial von der Nadelwelle 5 absteht. Der Greiferantriebsexzenter 10 wirkt über eine Pleuelstange 15, eine Schwingwelle 16 und eine Pendelplatte 17 mit der Greiferstange 6 zusammen. Die Greiferantriebsnut 11 nimmt eine Rolle 18 auf, welche an dem dem Schwinggreifer 2 abgewandten Ende der Greiferstange 6 drehbar gelagert ist.

Die Pleuelstange 15 ist mittels einer Verbindungslasche 19 an einen Betätigungsarm 20 angelenkt, welcher radial von der Schwingwelle 16 absteht. Weiterhin ist die Pleuelstange 15 an einen Führungsarm 21 angelenkt, welcher radial von der Nadelwelle 5 absteht und auf derselben drehbar gelagert ist. Die Pendelplatte 17 ist an einem Tragarm 22 aufgehängt, welcher radial zur Nadelwelle 5 orientiert ist, sich bezüglich der Nadelwelle 5 frei drehen kann und einen zur Nadelwelle 5 parallelen Tragbolzen 23 aufweist, der in einer entsprechenden Bohrung der Pendelplatte 17 aufgenommen ist.

Die Nadelwelle 5 und die Schwingwelle 16 erstrecken sich parallel zueinander in Maschinenlängsrichtung und sind im Maschinengehäuse 24 drehbar gelagert. Die Steuerwalze 8 und deren Zahnrad 14 sind auf der Schwingwelle 16 zwischen der Pendelplatte 17 und der Verbindungslasche 19 drehbar gelagert. Die Ausgangswelle 12 des Gleichstrommotors 3 verläuft parallel zur Nadelwelle 5 und zur Schwingwelle 16.

Die chirurgischen Nähmaschinen dienen jeweils zum Vernähen von Geweberändern miteinander, beispielweise von zwei Geweberändern A, B. Dabei oszilliert die Bogennadel 1 in Richtung der Pfeile 25, 26 hin und her, schwingt der zur Stichbildung mit der Bogennadel 1 zusammenwirkende Schwinggreifer 2 in Richtung der Pfeile 27, 28 hin und her und werden die Geweberänder A, B gemeinsam schrittweise in Richtung des Pfeils 29 vorgeschoben, so daß eine Einfaden-Überwendlichnaht C entsteht. Die Greiferstange 6 ist in einer Kugel 30 axial verschieblich und drehbar gelagert, welche ihrerseits in der Vorderwand 31 des Maschinengehäuses 24 universell beweglich gelagert ist.

Die chirurgischen Nähmaschinen sind jeweils mit einer Pinzette 32 versehen, um die Geweberänder A, B im Bereich der sich quer durch dieselben erstreckenden Bewegungsbahn der Bodennadel 1 abzustützen, wenn sie miteinander vernäht werden. Die Pinzette 32 schließt und öffnet im Takt der Bewegungen der Bogennadel 1 und weist einen rechten Schenkel 33 sowie einen linken Schenkel 34 auf. Die beiden Pinzettenschenkel 33, 34 sind beiderseits eines von der Maschinengehäusevorderwand 31 nach vorne abstehenden, stationären Mittelarms 35 angeordnet und vor jedem Einstechen der Bogennadel 1 in die Geweberänder A, B in eine Schließstellung sowie nach dem anschließenden Austreten der Bogennadel 1 aus den Geweberändern A, B in eine Öffnungsstellung bewegbar, um in der Schließstellung die beiden Geweberänder A, B zusammenzuhalten, nämlich jeweils den einen Geweberand A gegen die eine Seite des Mittelarms 35 bzw. den anderen Geweberand B gegen die andere Seite des Mittelarms 35 zu pressen, und in der Öffnungsstellung die Geweberänder A, B freizugeben.

Der rechte Pinzettenschenkel 33 steht am vorderen Ende einer hohlen Außenwelle 36 im wesentlichen radial ab, der linke Pinzettenschenkel 34 am vorderen Ende einer hohlen Innenwelle 37, welche sich durch die hohle Außenwelle 36 hindurch erstreckt und durch welche hindurch die Nadelwelle 5 verläuft. Die hohle Innenwelle 37 ist auf der Nadelwelle 5 drehbar gelagert, die hohle Außenwelle 36 auf der Innenwelle 37. Die Nadelwelle 5 ist in der Maschinengehäusevorderwand 31 über die hohle Innenwelle 37 und die hohle Außenwelle 36 drehbar gelagert. Der Tragarm 22 für die Pendelplatte 17 zur Bewegung des Schwinggreifers 2 ist innen neben der Maschinengehäusevorderwand 31 auf der Außenwelle 36 drehbar gelagert.

Der rechte Pinzettenschenkel 33, der linke Pinzettenschenkel 34 und der stationäre Mittelarm 35 weisen jeweils am freien Ende eine plattenförmige Zunge 38 zur Anlage an dem einen Geweberand A bzw. an dem anderen Geweberand B bzw. an beiden Geweberändern A, B auf, welche mit einer Durchgangsöffnung 39 für die Bogennadel 1 versehen ist. Jede Nadeldurchgangsöffnung 39 ist als eine zur Nadelwelle 5 hin offene Ausnehmung ausgebildet.

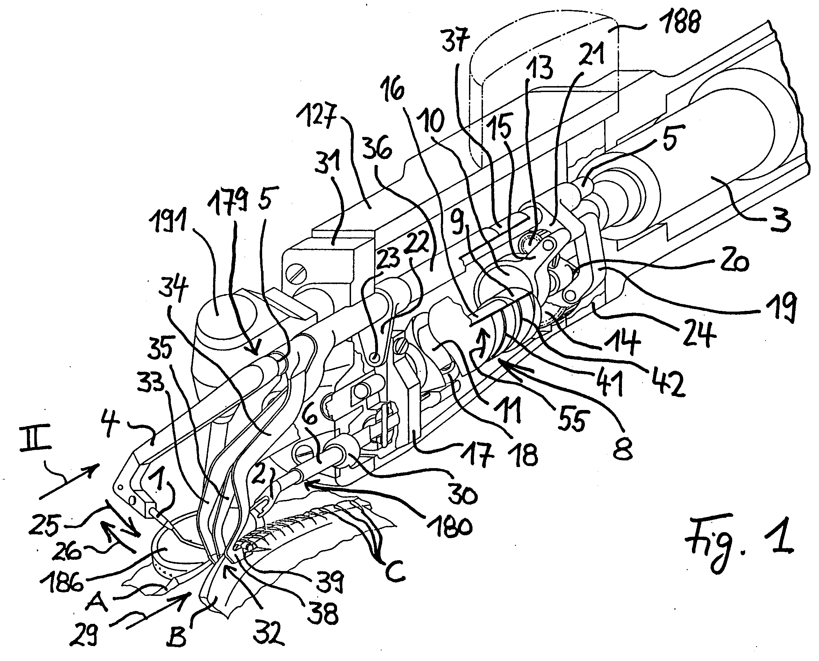

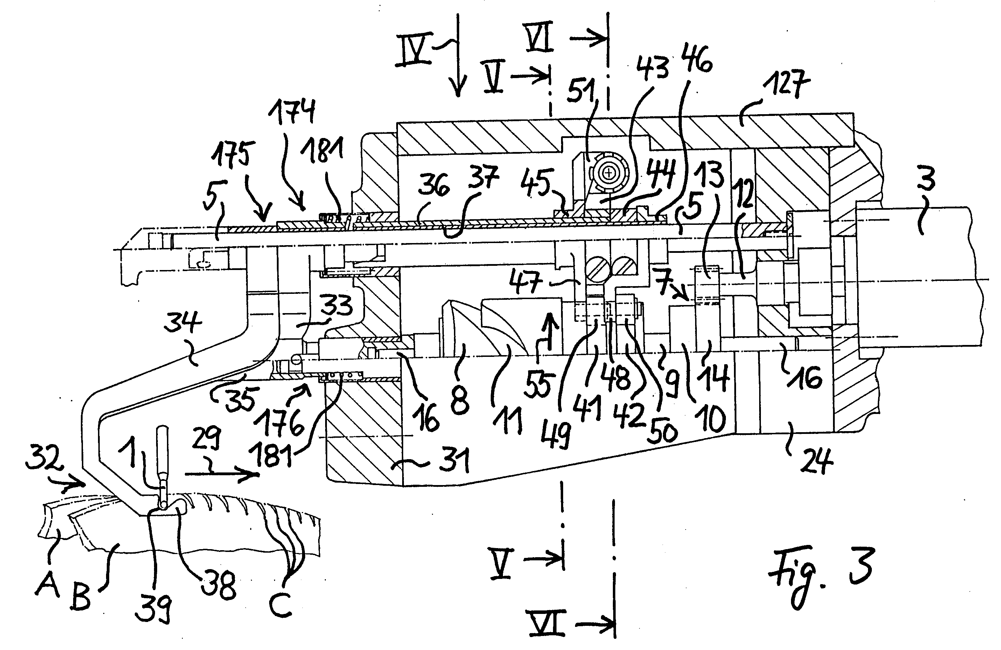

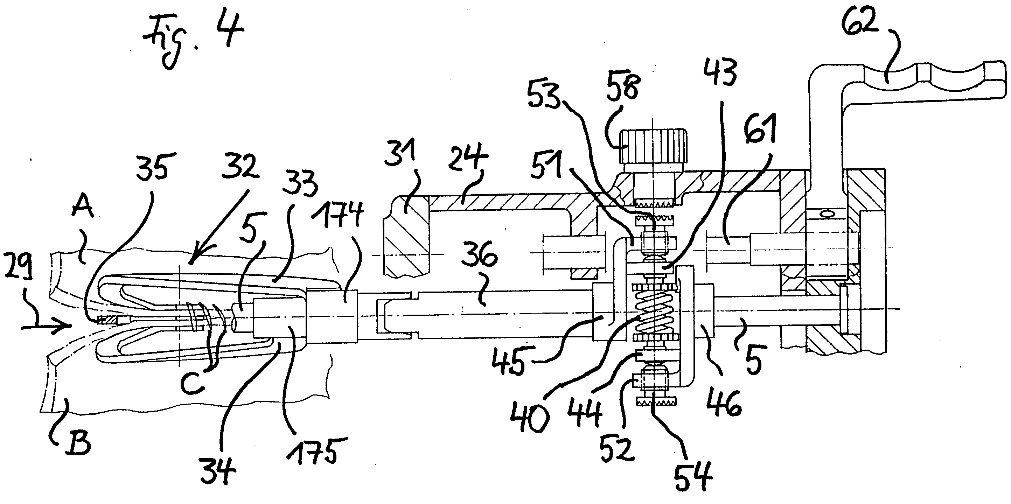

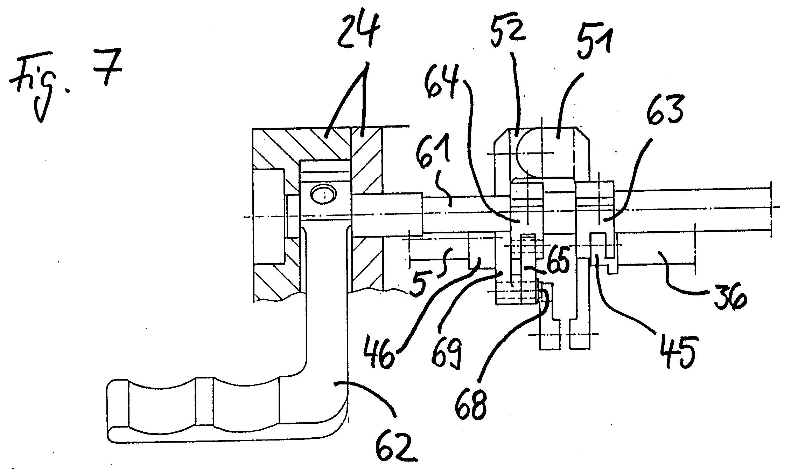

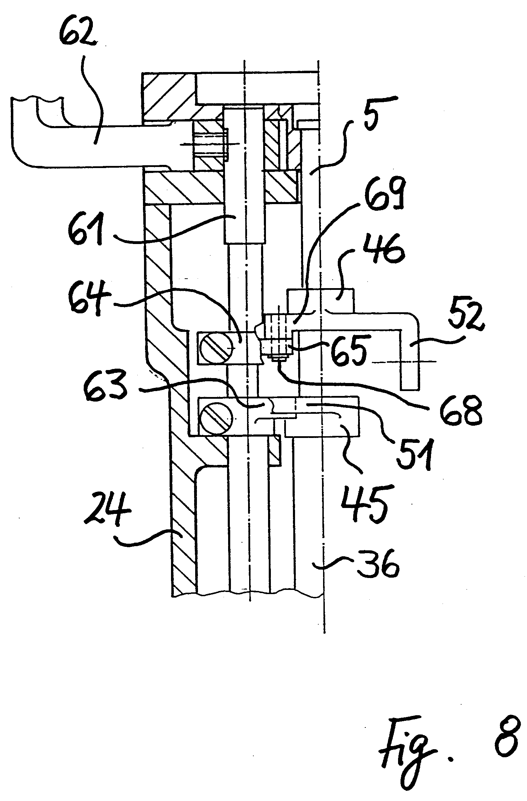

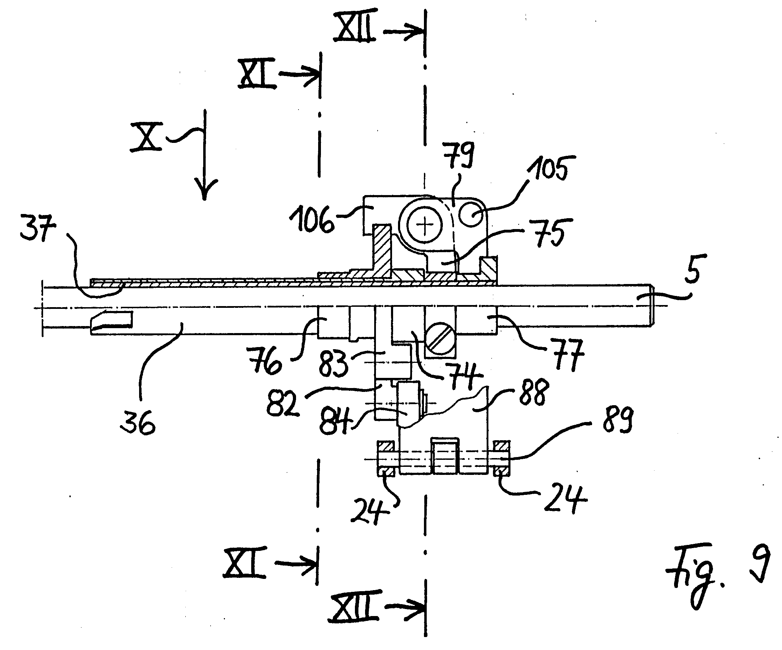

Bei der Pinzette 32 gemäß Fig. 1 bis 8 sind der rechte Pinzettenschenkel 33 und der linke Pinzettenschenkel 34 durch eine gemeinsame Schraubendruckfeder 40 in die Schließstellung belastet und durch je einen an der Steuerwalze 8 ausgebildeten Steuernocken 41 bzw. 42 entgegen der Wirkung der Federbelastung in die Öffnungsstellung bewegbar. Die Schraubendruckfeder 40 ist zwischen zwei ersten Betätigungshebeln 43, 44 für den rechten Pinzettenschenkel 33 bzw. den linken Pinzettenschenkel 34 angeordnet, welche jeweils am hinteren Ende der Außenwelle 36 bzw. der Innenwelle 37 im wesentlichen radial abstehen und über einen zweiten Betätigungshebel 45 bzw. 46 mit dem Steuernocken 41 für den rechten Pinzettenschenkel 33 bzw. mit dem Steuernocken 42 für den linken Pinzettenschenkel 34 zusammenwirken.

Die beiden zweiten Betätigungshebel 45, 46 sind jeweils auf der Außenwelle 36 bzw. auf der Innenwelle 37 drehbar gelagert und mit einem im wesentlichen radial abstehenden Arm 47 bzw. 48 zur Anlage am zugehörigen Steuernocken 41 bzw. 42 über eine am freien Ende drehbar gelagerte Rolle 49 bzw. 50 sowie mit einem im wesentlichen radial abstehenden Arm 51 bzw. 52 versehen, welcher sich auf der der Schraubendruckfeder 40 abgewandten Seite des zugehörigen ersten Betätigungshebels 43 bzw. 44 erstreckt und in welchem ein Druckstift 53 bzw. 54 verschraubbar ist, welcher außen am zugehörigen ersten Betätigungshebel 43 bzw. 44 anliegt.

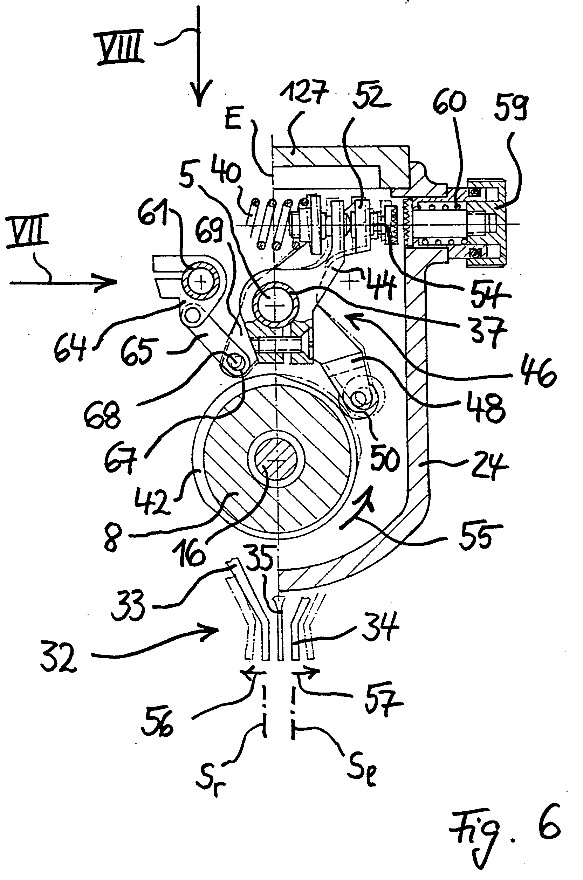

In Fig. 1 bis 8 befinden sich die Schenkel 33, 34 der Pinzette 32 in der Schließstellung, aus welcher sie beim Umlauf der Steuerwalze 8 in Richtung des Pfeils 55 durch deren Steuernocken 41, 42 in die in Fig. 6 mit strichpunktierten Linien angedeutete Öffnungsstellung verschwenkt werden, jeweils in Richtung des Pfeils 56 bzw. 57. Die Steuernocken 41, 42 sind so ausgebildet, daß die Pinzettenschenkel 33, 34 dabei identische Bewegungen in Richtung der Pfeile 56, 57 ausführen, wobei auch die zweiten Betätigungshebel 45, 46 und die ersten Betätigungshebel 43, 44 um identische Winkel in einander entgegengesetzten Richtungen verschwenken. Diejenige Position Sᵣ bzw. Sₗ, welche der rechte Pinzettenschenkel 33 bzw. der linke Pinzettenschenkel 34 gemäß Fig. 6 in der Schließstellung einnimmt, läßt sich mit Hilfe des zugehörigen Druckstiftes 53 bzw. 54 verändern und einstellen. Beim Hineinschrauben des Druckstiftes 53 im Arm 51 des zweiten Betätigungshebels 45 des rechten Pinzettenschenkels 33 vergrößert sich dessen Abstand vom stationären Mittelarm 35, um sich zu verringern, wenn der Druckstift 53 im Arm 51 herausgeschraubt wird. Das Verschrauben des Druckstiftes 54 im Arm 52 des zweiten Betätigungshebels 46 des linken Pinzettenschenkels 34 bewirkt dasselbe in bezug auf dessen Abstand vom stationären Mittelarm 35.

Die beiden Druckstifte 53, 54 sind jeweils mittels eines Betätigungsknopfes 58 bzw. 59 verschraubbar, welcher gegen die Wirkung einer Schraubendruckfeder 60 gedrückt werden muß, um ihn mit dem Druckstift 53 bzw. 54 zu kuppeln.

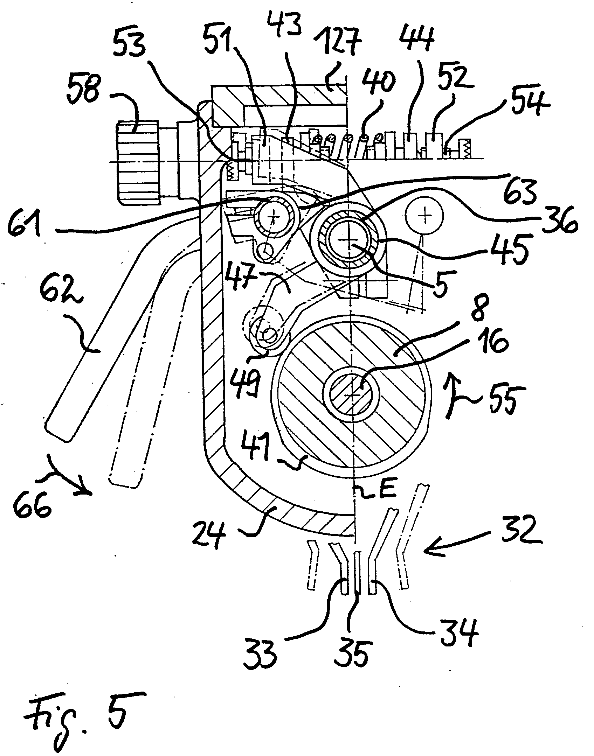

Um die Geweberänder A, B vor dem Vernähen in die chirurgische Nähmaschine nach Fig. 1 bis 8 einsetzen und nach dem Vernähen daraus entnehmen zu können, muß die Pinzette 32 gelüftet werden. Dieses geschieht mit Hilfe einer zur Außenwelle 36 und Innenwelle 37 paralleler, Stellwelle 61, welche im Maschinengehäuse 24 drehbar gelagert ist und einen aus dem Maschinengehäuse 24 nach außen ragenden Handhebel 62, eine im wesentlichen radial abstehende Nase 63 und einen im wesentlichen radial abstehenden Arm 64 aufweist. Die Nase 63 wirkt mit dem zweiten Betätigungshebel 45 des rechten Pinzettenschenkels 33 unmittelbar zusammen, der Arm 64 mit dem zweiten Betätigungshebel 46 des linken Pinzettenschenkels 34 über eine Drucklasche 65, um beim Verschwenken des Handhebels 62 aus der in Fig. 5 mit ausgezogenen Linien wiedergegebenen Ruhestellung in die dort mit strichpunktierten Linien angedeutete Lüftungsstellung und dem entsprechenden Drehen der Stellwelle 61 in Richtung des Pfeils 66 die beiden Pinzettenschenkel 33, 34 entgegen der Wirkung der Schraubendruckfeder 40 auseinanderzuspreizen, so daß sie die in Fig. 5 mit strichpunktierten Linien angedeutete Lüftungsstellung einnehmen. Die an den Arm 64 angelenkte Drucklasche 65 ist am anderen Ende mit einem Langloch 67 versehen, in welches ein Stift 68 an einem dritten im wesentlichen radialen Arm 69 des zweiten Betätigungshebels 46 des linken Pinzettenschenkels 34 eingreift. Das Langloch 67 ermöglicht das Hin- und Herschwenken des zweiten Betätigungshebels 46 bei umlaufendem zugehörigen Steuernocken 42 ohne Rückwirkung auf die Stellwelle 61, wenn deren Handhebel 62 sich in der Ruhestellung befindet.

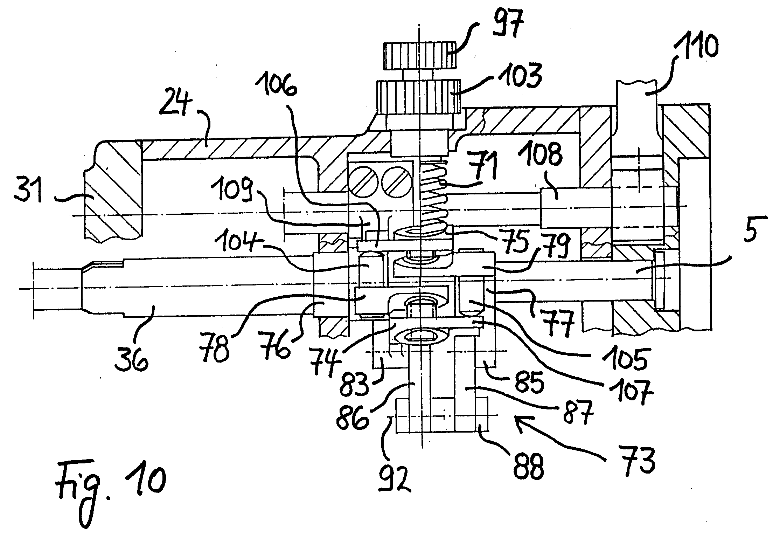

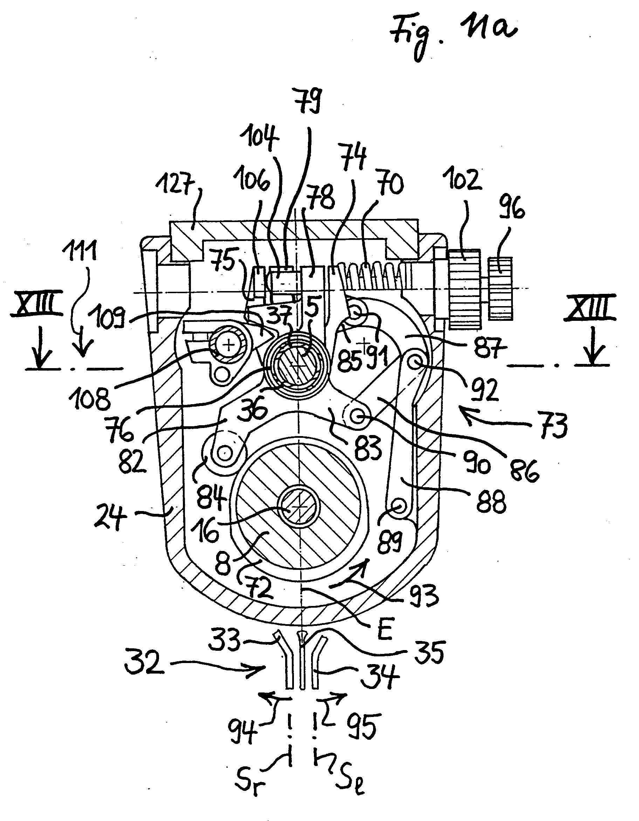

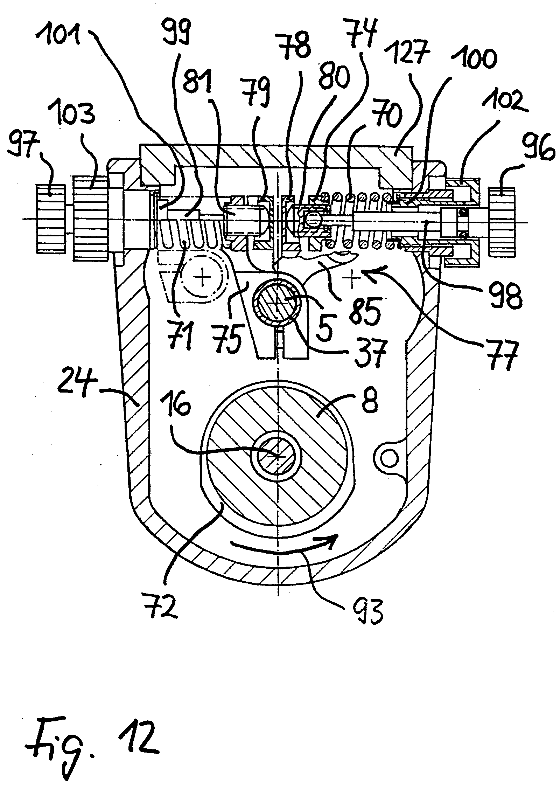

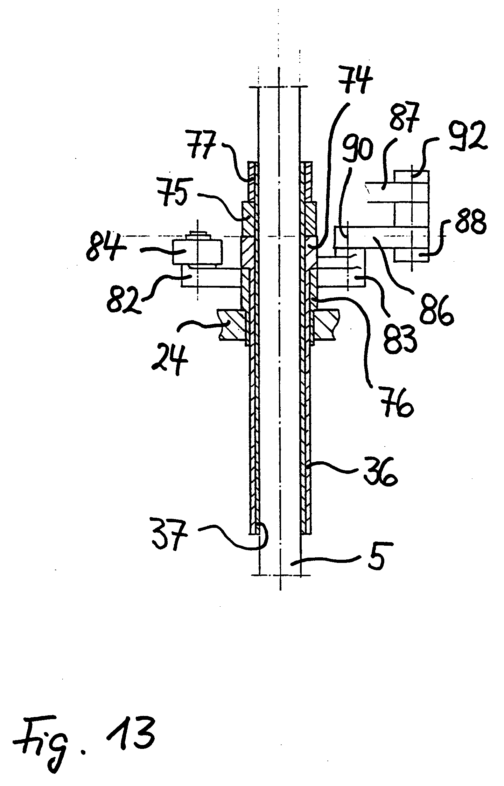

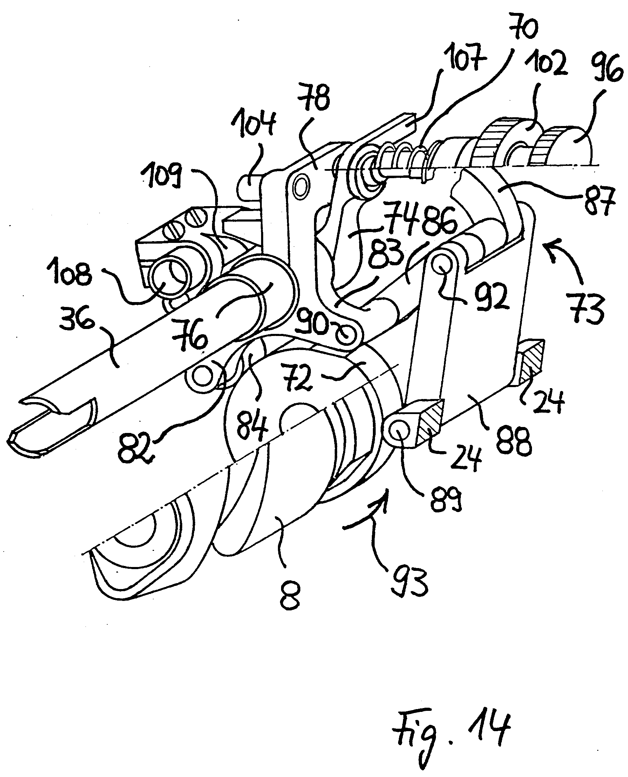

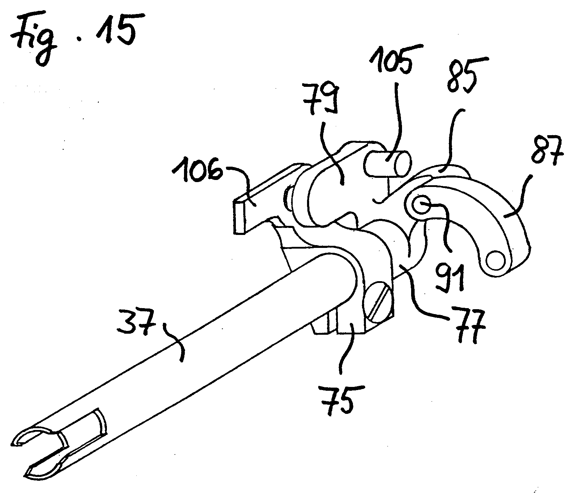

Bei der Pinzette 32 gemäß Fig. 9 bis 15 sind der rechte Pinzettenschenkel 33 und der linke Pinzettenschenkel 34 durch je eine Schraubendruckfeder 70 bzw. 71 in die Schließstellung belastet und durch nur einen an der Steuerwalze 8 ausgebildeten Steuernocken 72 entgegen der Wirkung der Federbelastung in die Öffnungsstellung bewegbar, wobei die beiden Pinzettenschenkel 33, 34 durch ein Gestänge 73 miteinander gekoppelt sind. Die Vorspannung der Schraubendruckfeder 70 des rechten Pinzettenschenkels 33 und dessen Position Sᵣ, welche er in der Schließstellung dann einnimmt, wenn sich keine Geweberänder in der chirurgischen Nähmaschine befinden, können unabhängig voneinander verändert und eingestellt werden, ebenso wie die Vorspannung der Schraubendruckfeder 71 des linken Pinzettenschenkels 34 und dessen Schließstellungsposition Sₗ. Es sind besondere Maßnahmen getroffen, so daß die Schwenkwinkel der Pinzettenschenkel 33, 34 zwischen der Schließstellung und der Öffnungsstellung von den eingestellten Schließstellungspositionen Sᵣ, Sₗ abhängen und um so größer sind, je größer die Abstände der Pinzettenschenkel 33, 34 in den Schließstellungspositionen Sᵣ, Sₗ vom stationären Mittelarm 35 sind. Dieses ist im Hinblick auf die Eigenschaften der miteinander zu vernähenden Geweberänder, insbesondere deren Elastizität, für das sichere und dennoch schonende Zusammenhalten der Geweberänder in der Schließstellung und für ihre vollständige Freigabe in der Öffnungsstellung der Pinzettenschenkel 33, 34 günstig.

Die beiden Schraubendruckfedern 70, 71 sind außerhalb von zwei ersten Betätigungshebeln 74, 75 für den rechten Pinzettenschenkel 33 bzw. den linken Pinzettenschenkel 34 angeordnet, welche jeweils am hinteren Ende der Außenwelle 36 bzw. der Innenwelle 37 im wesentlichen radial abstehen und über einen zweiten Betätigungshebel 76 bzw. 77 mit dem Steuernocken 72 bzw. dem Gestänge 73 zusammenwirken.

Die beiden zweiten Betätigungshebel 76, 77 sind jeweils auf der Außenwelle 36 bzw. auf der Innenwelle 37 drehbar gelagert und mit einem im wesentlichen radial abstehenden Arm 78 bzw. 79 versehen, welcher sich auf der der Schraubendruckfeder 70 des rechten Pinzettenschenkels 33 abgewandten Seite des zugehörigen ersten Betätigungshebels 74 bzw. auf der der Schraubendruckfeder 71 des linken Pinzettenschenkels 34 abgewandten Seite des zugehörigen ersten Betätigungshebels 75 erstreckt und an welchem ein Druckstift 80 bzw. 81 außen anliegt, der im zugehörigen ersten Betätigungshebel 74 bzw. 75 verschraubbar ist. Weiterhin weist der zweite Betätigungshebel 76 des rechten Pinzettenschenkels 33 zwei jeweils im wesentlichen radial abstehende Arme 82, 83 zur Anlage am Steuernocken 72 über eine am freien Ende drehbar gelagerte Rolle 84 bzw. für das Gestänge 73 auf. Auch der zweite Betätigungshebel 77 des linken Pinzettenschenkels 34 ist mit einem im wesentlichen radial abstehenden Arm 85 für das Gestänge 73 versehen.

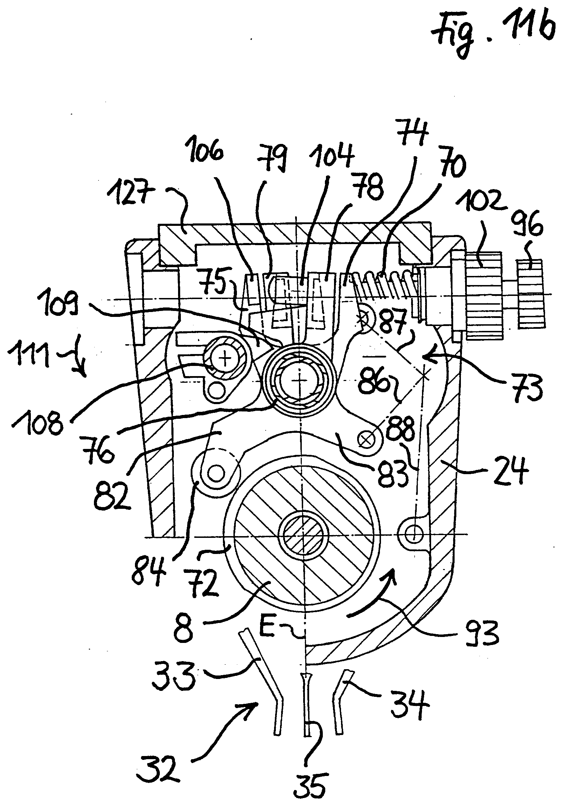

Die beiden Gestängearme 83, 85 sind jeweils über eine Verbindungslasche 86 bzw. 87 an das eine Ende eines Schwenkhebels 88 angelenkt, welcher am anderen Ende mit dem Maschinengehäuse 24 um eine zur Außenwelle 36 und Innenwelle 37 parallele Achse 89 verschwenkbar verbunden ist. Auch die Schwenkachse 90 bzw. 91 zwischen jeder Verbindungslasche 86 bzw. 87 und dem zugehörigen Gestängearm 83 bzw. 85 und die Schwenkachse 92 zwischen den beiden Verbindungslaschen 86, 87 und dem Schwenkhebel 88 verlaufen parallel zur Außenwelle 36 und Innenwelle 37. Die Gestängearme 83, 85 und die Verbindungslaschen 86, 87 sowie der Schwenkhebel 88 bilden das Gestänge 73. Sie sind so ausgebildet und angeordnet, daß die beiden zweiten Betätigungshebel 76, 77 immer nur spiegelbildliche Positionen bezüglich der Symmetrieebene E einnehmen können, in welcher sich der stationäre Mittelarm 35 erstreckt, wie Fig. 11a und 11b veranschaulichen.

In Fig. 9 bis 11a und 12 bis 15 befinden sich die Schenkel 33, 34 der Pinzette 32 in der Schließstellung, aus welcher sie beim Umlauf der Steuerwalze 8 in Richtung des Pfeils 93 in die Öffnungsstellung gemaß Fig. 11b verschwenkt werden, jeweils in Richtung des Pfeils 94 bzw. 95 in Fig. 11a. Dabei verschwenkt der Steuernocken 72 der Steuerwalze 8 den dem rechten Pinzettenschenkel 33 zugeordneten zweiten Betätigungshebel 76, welcher seinerseits den dem linken Pinzettenschenkel 34 zugeordneten zweiten Betätigungshebel 77 über das Gestänge 73 mitnimmt, so daß er um denselben Winkel in der entgegengesetzten Richtung verschwenkt. Die beiden zweiten Betätigungshebel 76, 77 nehmen jeweils den zugehörigen ersten Betätigungshebel 74 bzw. 75 mit, und zwar über den Druckstift 80 bzw. 81, welcher im ersten Betätigungshebel 74 bzw. 75 zur Veränderung und Einstellung der Schließstellungsposition Sᵣ bzw. Sₗ des rechten Pinzettenschenkels 33 bzw. des linken Pinzettenschenkels 34 verschraubbar ist, wobei das Hineinschrauben den Abstand des rechten Pinzettenschenkels 33 bzw. des linken Pinzettenschenkels 34 vom stationären Mittelarm 35 vergrößert und das Herausschrauben den Abstand verringert. Die beiden Druckstifte 80, 81 sind jeweils mittels eines Betätigungsknopfes 96 bzw. 97 mit einem Schaft 98 bzw. 99 verschraubbar, welcher am Druckstift 80 bzw. 81 angreift.

Die beiden Schraubendruckfedern 70, 71 stützen sich jeweils an dem dem zugehörigen ersten Betätigungshebel 74 bzw. 75 abgewandten Ende an einer Schraubhülse 100 bzw. 101 ab, welche koaxial zum Schaft 98 bzw. 99 des Betätigungsknopfes 96 bzw. 97 für den Druckstift 80 bzw. 81 des ersten Betätigungshebels 74 bzw. 75 angeordnet und mittels eines Betätigungsknopfes 102 bzw. 103 Im Maschinengehäuse 24 verschraubbar ist, um die Vorspannung der Schraubendruckfeder 70 bzw. 71 einzustellen.

Die beiden zweiten Betätigungshebel 76, 77 des rechten Pinzettenschenkels 33 bzw. des linken Pinzettenschenkels 34 sind jeweils mit einem stiftförmigen Anschlag 104 bzw. 105 für den ersten Betätigungshebel 75 bzw. 74 des linken Pinzettenschenkels 34 bzw. des rechten Pinzettenschenkels 33 versehen. Die Anschläge 104, 105 erstrecken sich quer zur Außenwelle 36 und Innenwelle 37 jeweils vom oberen Arm 78 bzw. 79 des zweiten Betätigungshebels 76 bzw. 77 weg auf eine seitliche Zunge 106 bzw. 107 des ersten Betätigungshebels 75 bzw. 74 zu. In der Schließstellung der Pinzettenschenkel 33, 34 kommen die Anschläge 104, 105 jeweils an der zugehörigen Zunge 106 bzw. 107 zur Anlage, um den gegenseitigen Abstand des zweiten Betätigungshebels 76 des rechten Pinzettenschenkels 33 und des ersten Betätigungshebels 75 des linken Pinzettenschenkels 34 bzw. des zweiten Betätigungshebels 77 des linken Pinzettenschenkels 34 und des ersten Betätigungshebels 74 des rechten Pinzettenschenkels 33 zu begrenzen.

In Verbindung mit dem Gestänge 73 hat dieses zur Folge, daß derjenige Winkel, den der obere Arm 78 bzw. 79 jedes zweiten Betätigungshebels 76 bzw. 77 mit der Symmetrieebene E in der Schließstellung der Pinzettenschenkel 33, 34 einschließt, und somit auch derjenige Winkel, den der mit dem Steuernocken 72 zusammenwirkende Arm 82 des zweiten Betätigungshebels 76 des rechten Pinzettenschenkels 33 mit der Symmetrieebene E in der Schließstellung der Pinzettenschenkel 33, 34 einschließt, um so kleiner ist, je größer die Abstände der Pinzettenschenkel 33, 34 in den Schließstellungspositionen Sᵣ, Sₗ von der Symmetrieebene E sind, je weiter also die Druckstifte 80, 81 in den ersten Betätigungshebeln 74, 75 nach innen geschraubt sind. Der Abstand der Rolle 84 des mit dem Steuernocken 72 zusammenwirkenden Arms 82 von der Drehachse der Steuerwalze 8 verringert sich entsprechend, so daß der Hub, um welchen der Steuernocken 72 die Rolle 84 beim Umlauf der Steuerwalze 8 von deren Drehachse wegbewegen kann, sich entsprechend vergrößert, somit auch der Schwenkwinkel jedes Pinzettenschenkels 33 bzw. 34 beim Pinzettenöffnen.

In Fig. 9 bis 15 ist die Pinzette 32 in demjenigen Zustand veranschaulicht, wobei die Druckstifte 80, 81 am weitesten eingeschraubt sind, so daß die Pinzettenschenkel 33, 34 jeweils den größten Abstand vom stationären Mittelarm 35 haben, wenn sie sich in der Schließstellung gemäß Fig. 11a befinden, in welcher die Rolle 84 des zweiten Betätigungshebels 76 des rechten Pinzettenschenkels 33 an dem tieferen Umfangsabschnitt des Steuernockens 72 anliegt, so daß der gesamte radiale Höhenunterschied zwischen dem tieferen Umfangsabschnitt und dem höheren Umfangsabschnitt des Steuernockens 72 zur Verfügung steht, um den zweiten Betätigungshebel 76 beim Umlauf der Steuerwalze 8 in die Stellung gemäß Fig. 11b zu verschwenken. Wenn dünnere Geweberänder miteinander vernäht werden sollen, dann werden die Druckstifte 80, 81 in den ersten Betätigungshebeln 74, 75 nach außen geschraubt, um die Schließstellungspositionen Sᵣ, Sₗ der Pinzettenschenkel 33, 34 neu einzustellen und ihre Schließstellungsabstände vom stationären Mittelarm 35 entsprechend zu verringern. Dabei wird die Rolle 84 des zweiten Betätigungshebels 76 entsprechend weit vom tieferen Umfangsabschnitt des Steuernockens 72 wegbewegt, so daß derjenige Winkel, um welchen der höhere Umfangsabschnitt des Steuernockens 72 den Zweiten Betätigungshebel 76 beim Umlauf der Steuerwalze 8 verschwenkt, sich entsprechend verringert.

Zum Lüften der Pinzette 32 ist eine Stellwelle 108 vorgesehen, welche sich lediglich dadurch von der Stellwelle 61 der chirurgischen Nähmaschine nach Fig. 1 bis 8 unterscheidet, daß sie nur eine im wesentlichen radial abstehende Nase 109 zum Verschwenken des zweiten Betätigungshebels 76 des rechten Pinzettenschenkels 33 aufweist. Wenn die Stellwelle 108 mittels ihres Handhebels 110 in Richtung des Pfeils 111 gedreht wird, dann verschwenkt sie den zweiten Betätigungshebel 76 unmittelbar und den zweiten Betätigungshebel 77 des linken Pinzettenschenkels 34 über das Gestänge 73, um die beiden Pinzettenschenkel 33, 34 entgegen der Wirkung der Schraubendruckfedern 70, 71 auseinanderzuspreizen.

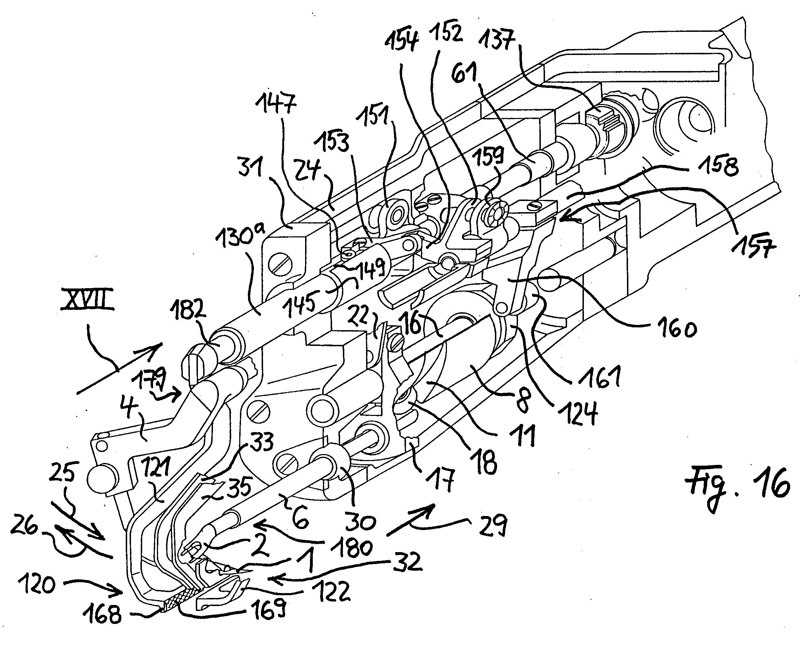

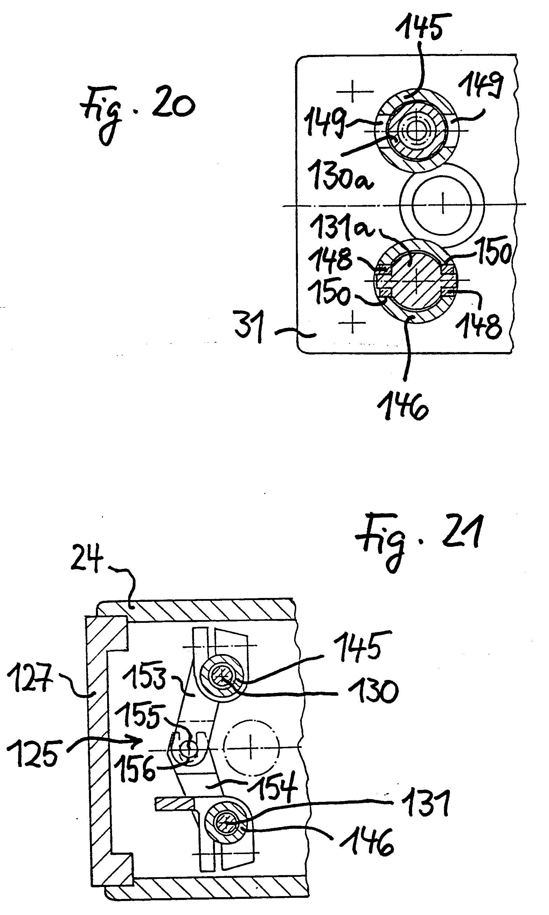

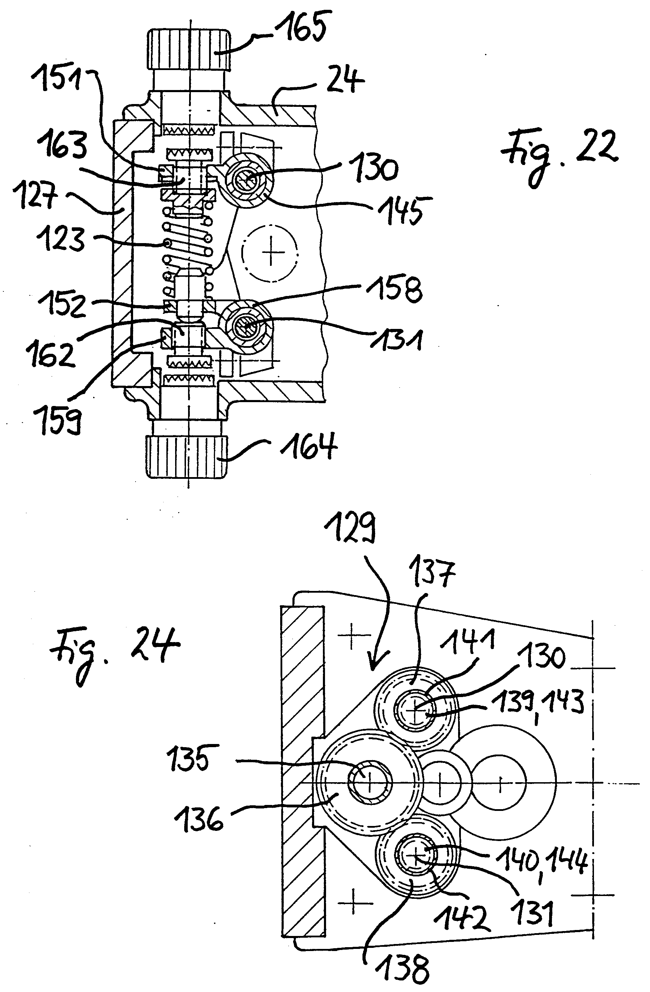

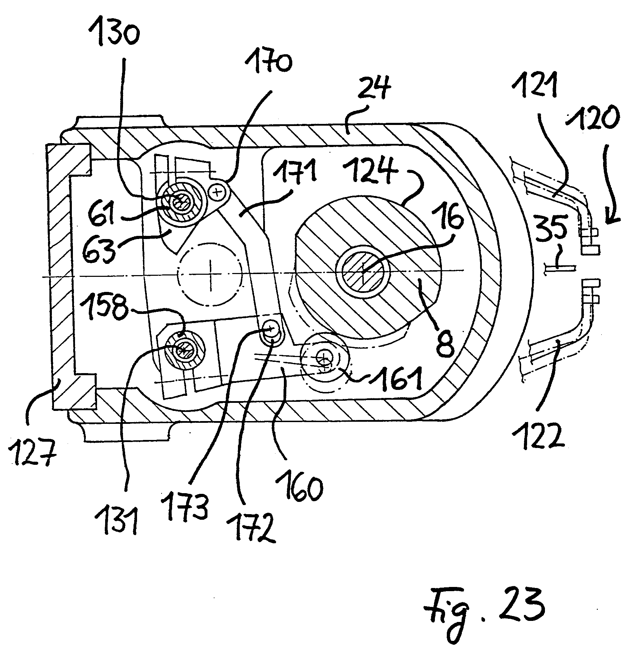

Für den schrittweisen Vorschub der Geweberänder A, B in Richtung des Pfeils 29 ist bei der chirurgischen Nähmaschine nach Fig. 16 bis 24 eine Vorschubzange 120 vorgesehen, welche im Takt des Öffnens und Schließens der Pinzette 32 arbeitet und einen rechten Schenkel 121 sowie einen linken Schenkel 122 aufweist. Die Vorschubzange 120 führt zweierlei Bewegungen aus, nämlich einerseits eine Schließ - und Öffnungsbewegung sowie andererseits eine Vorschub- und Rückbewegung, und die beiden Zangenschenkel 121, 122 sind demgemäß in zweifacher Weise bewegbar, nämlich erstens zwischen einer Schließstellung und einer Öffnungsstellung, um die Geweberänder A, B zu erfassen bzw. freizugeben, und zweitens zwischen einer Ausgangsstellung sowie einer Endstellung in der Vorschubrichtung 29 und entgegen der Vorschubrichtung 29, um die Geweberänder A, B zu transportieren bzw. wieder die Ausgangsstellung zur Durchführung des nächsten Vorschubschrittes einzunehmen. Das Schließen und Öffnen der Vorschubzange 120, das Vor- und Rücklaufen der Vorschubzange 120 und das Schließen sowie Öffnen der Pinzette 32 sind so aufeinander abgestimmt, daß die Zangenschenkel 121, 122 sich während jeder Öffnungsperiode der Pinzette 32 in der Schließstellung aus der Ausgangsstellung in die Endstellung und während jeder Schließperiode der Pinzette 32 in der Öffnungsstellung aus der Endstellung in die Ausgangsstellung bewegen.

Der rechte Zangenschenkel 121 und der linke Zangenschenkel 122 sind durch eine gemeinsame Schraubendruckfeder 123 in die Schließstellung belastet und durch einen an der Steuerwalze 8 ausgebildeten Steuernocken 124 entgegen der Wirkung der Federbelastung in die Öffnungsstellung bewegbar, wobei die beiden Zangenschenkel 121, 122 durch ein Gestänge 125 miteinander gekoppelt sind. Zur gemeinsamen Bewegung der Zangenschenkel 121, 122 in der Vorschubrichtung 29 und entgegen der Vorschubrichtung 29 ist ein umsteuerbarer elektrischer Schrittmotor 126 vorgesehen.

Der elektrische Schrittmotor 126 ist auf einem Maschinengehäusedeckel 127 angeordnet und treibt über ein Winkelgetriebe 128 sowie ein Verteilergetriebe 129 zwei Spindeln 130, 131 an, welche jeweils dem rechten Zangenschenkel 121 bzw. dem linken Zangenschenkel 122 zugeordnet sind. Das Winkelgetriebe 128 weist zwei miteinander kämmende Kegelräder 132, 133 auf, welche jeweils mit der Ausgangswelle 134 des elektrischen Schrittmotors 126 bzw. mit der Eingangswelle 135 des Verteilergetriebes 129 verbunden sind. Letzteres setzt sich aus einem mit der Eingangswelle 135 verbundenen Eingangszahnrad 136 und zwei Ausgangszahnrädern 137, 138 zusammen, welche jeweils mit dem Eingangszahnrad 136 kämmen und mit der Spindel 130 des rechten Zangenschenkels 121 bzw. mit der Spindel 131 des linken Zangenschenkels 122 zusammenwirken.

Die beiden Spindeln 130, 131 sind jeweils parallel zur Außenwelle 36 und Innenwelle 37 der Pinzette 32 sowie axial beweglich angeordnet und am hinteren Ende mit einem Außengewinde 139 bzw. 140 versehen. Sie erstrecken sich jeweils durch einen im Maschinengehäuse 24 drehbar, aber axial unbeweglich gelagerten Ring 141 bzw. 142 mit einem korrespondierenden Innengewinde 143 bzw. 144, welcher koaxial zum einen bzw. anderen Ausgangszahnrad 137 bzw. 138 des Verteilergetriebes 129 angeordnet und einstückig mit demselben ausgebildet ist. Wegen der gegenseitigen Verschraubbarkeit jeder Spindel 130 bzw. 131 und des zugehörigen Ringes 141 bzw. 142 infolge des Ineinandergreifens ihrer Außen- und Innengewinde 139, 143 bzw. 140, 144 bewirkt jedes Hin- und Herdrehen der Ringe 141, 142 durch den elektrischen Schrittmotor 126 ein entsprechendes axiales Hin- und Herschieben der Spindeln 130, 131. An deren vorderen Enden steht jeweils der rechte Zangenschenkel 121 bzw. der linke Zangenschenkel 122 im wesentlichen radial ab.

Zur Bewegung der beiden Zangenschenkel 121, 122 in die Schließstellung und in die Öffnungsstellung sind die beiden Spindeln 130, 131 hin- und herschwenkbar, und zwar mittels zweier hin- und herdrehbarer Schwenkhülsen 145, 146. In den Schwenkhülsen 145, 146 ist jeweils die eine Spindel 130 bzw. die andere Spindel 131 zwar axial verschiebbar, aber nicht drehbar. Dazu weist jede Spindel 130 bzw. 131 zwei einander diametral gegenüberliegende, um je eine radiale Achse drehbare Rollen 147 bzw. 148 auf, welche in zwei korrespondierenden Längsschlitzen 149 bzw. 150 der zugehörigen Schwenkhülse 145 bzw. 146 aufgenommen sind.

Die beiden Schwenkhülsen 145, 146 sind jeweils mit einem im wesentlichen radial abstehenden Betätigungshebel 151 bzw. 152 versehen. Zwischen den beiden Betätigungshebeln 151, 152 ist die Schraubendruckfeder 123 angeordnet. Weiterhin weist jede Schwenkhülse 145 bzw. 146 einen im wesentlichen radial abstehenden Arm 153 bzw. 154 auf, welcher am freien Ende mit einem Querstift 155 bzw. mit einer langlochartigen Ausnehmung 156 versehen ist. Die Arme 153, 154 greifen mittels des Querstiftes 155 und der Ausnehmung 156 ineinander und bilden das Gestänge 125, welches gewährleistet, daß die beiden Schwenkhülsen 145, 146 und Spindeln 130, 131 und Zangenschenkel 121, 122 immer nur um identische Winkel in einander entgegengesetzten Richtungen verschwenken können.

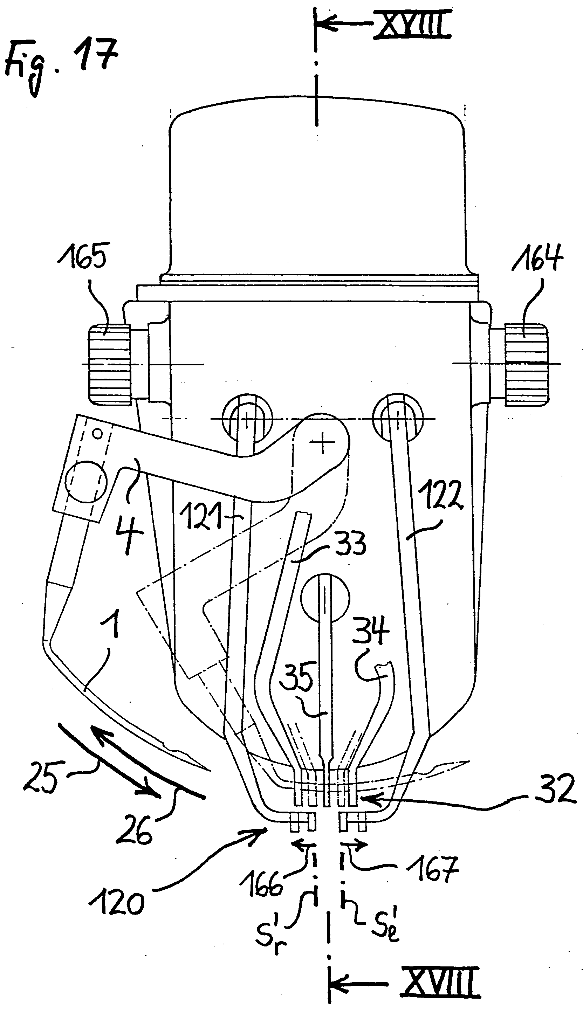

Der dem linken Zangenschenkel 122 zugeordnete Betätigungshebel 152 wirkt über einen zweiten Betätigungshebel 157 mit dem Steuernocken 124 zusammen. Der zweite Betätigungshebel 157 weist eine rohrförmige Nabe 158 auf, welche im Maschinengehäuse 24 drehbar gelagert ist und durch welche sich die Spindel 131 des linken Zangenschenkels 122 erstreckt. Weiterhin ist der zweite Betätigungshebel 157 mit zwei Armen 159, 160 zum Zusammenwirken mit dem ersten Betätigungshebel 152 bzw. mit dem Steuernocken 124 über eine am freien Ende drehbar gelagerte Rolle 161 versehen. Die Arme 159, 160 stehen im wesentlichen radial von der Nabe 158 ab. Der mit dem ersten Betätigungshebel 152 zusammenwirkende Arm 159 ist auf der der Schraubendruckfeder 123 abgewandten Seite des ersten Betätigungshebels 152 angeordnet und wirkt mit demselben über einen Druckstift 162 zusammen, welcher außen am ersten Betätigungshebel 152 anliegt und im Arm 159 verschraubbar ist, um die aus Fig. 17 ersichtlichen Schließstellungspositionen S'ᵣ, S'ₗ der Zangenschenkel 121, 122 zu verändern und einzustellen.

Auch die Vorspannung der Schraubendruckfeder 123 kann verändert und eingestellt werden. Dazu wirkt die Schraubendruckfeder 123 mit dem dem rechten Zangenschenkel 121 zugeordneten Betätigungshebel 151 über einen in demselben verschraubbaren Druckstift 163 zusammen. Wenn der Druckstift 163 im Betätigungshebel 151 nach innen geschraubt wird, erhöht sich die Vorspannung der Schraubendruckfeder 123. Wird er nach außen geschraubt, dann verringert sich die Vorspannung.

Die beiden Druckstifte 162, 163 sind jeweils mittels eines Betätigungsknopfes 164 bzw. 165 verschraubbar, welcher gegen die Wirkung einer Schraubendruckfeder gedrückt werden muß, um ihn mit dem Druckstift 162 bzw. 163 zu kuppeln, analog den Betätigungsknöpfen 58, 59 der chirurgischen Nähmaschine gemäß Fig. 1 bis 8.

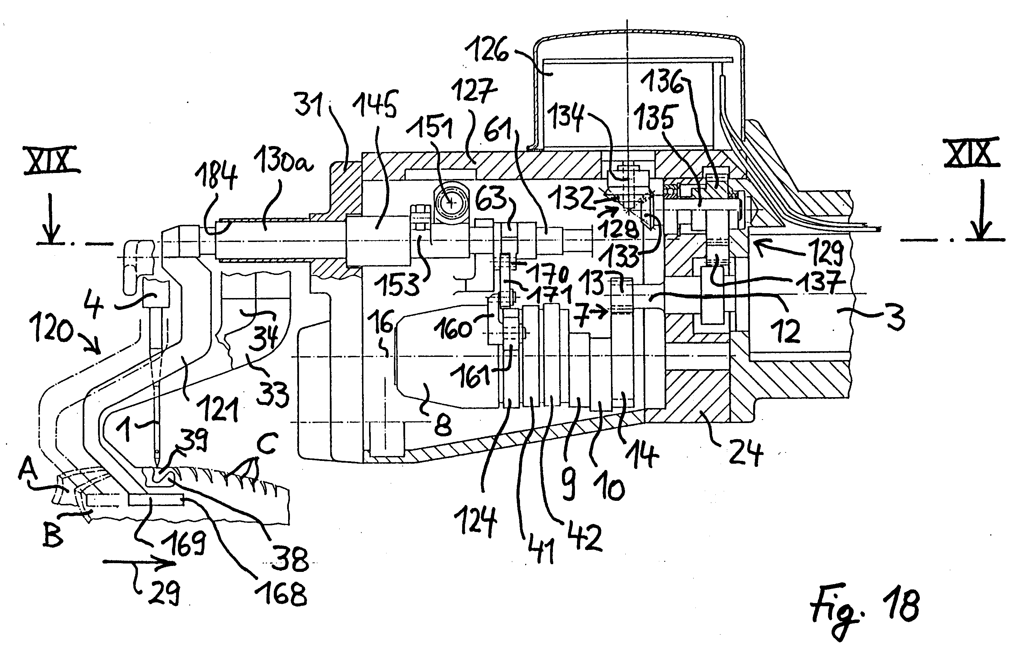

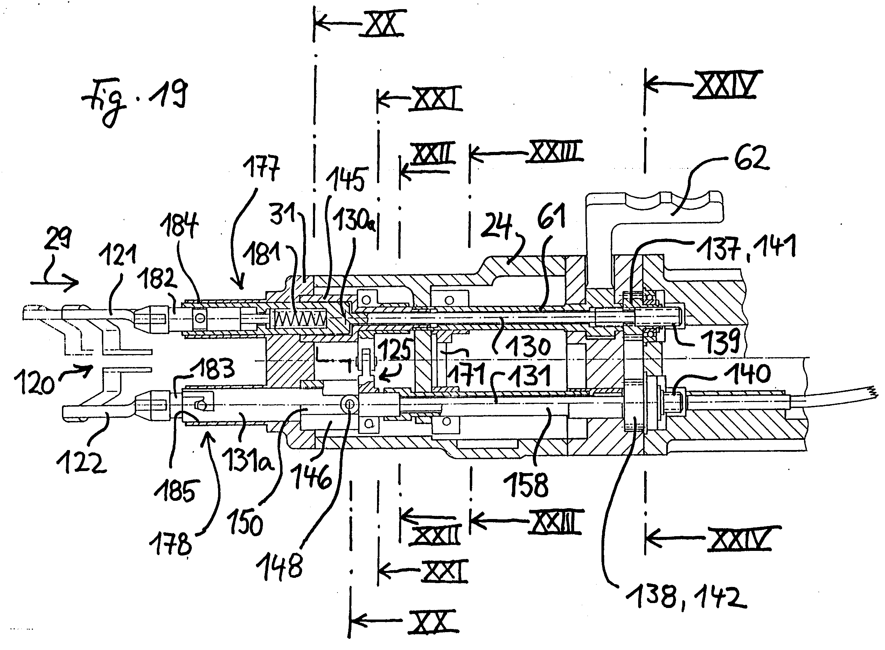

In Fig. 16 schwingt die Bogennadel 1 in Richtung des Pfeils 26, um aus den nicht dargestellten Geweberändern, welche gerade miteinander vernäht werden, auszutreten und in die in Fig. 17 mit ausgezogenen Linien wiedergegebene Umkehrstellung zu laufen. Der gabelförmige Schwinggreifer 2 bewegt sich dementsprechend in Richtung des Pfeils 28. Die Pinzette 32 ist geschlossen, ihre Schenkel 33, 34 befinden sich in der Schließstellung. Die Vorschubzange 120 ist geöffnet und bewegt sich entgegen der Vorschubrichtung 29 in die in Fig. 18, 19 mit strichpunktierten Linien angedeutete Ausgangsstellung, in welcher die spiegelbildlich zur Symmetrieebene E angeordneten und bewegbaren Zangenschenkel 121, 122 in die in Fig. 17 mit ausgezogenen Linien wiedergegebene Schließstellung schwenken, um die in Fig. 18 mit strichpunktierten Linien angedeuteten Geweberänder A, B unmittelbar jenseits der Pinzette 32, nämlich unterhalb der Zungen 38 der Pinzettenschenkel 33, 34 und auch des stationären Mittelarms 35, zu erfassen.

Nach dem Austritt der Bogennadel 1 aus den Geweberändern öffnet die Pinzette 32 und schwenken ihre Schenkel 33, 34 in die in Fig. 17 mit ausgezogenen Linien wiedergegebene Öffnungsstellung. Danach bewegt sich die Vorschubzange 120, deren Schenkel 121, 122 sich in der Schließstellung befinden, zusammen mit den erfaßten Geweberändern in der Vorschubrichtung 29 in die in Fig. 18, 19 mit ausgezogenen Linien wiedergegebene Endstellung, in welcher die Vorschubzange 120 wieder öffnet und ihre Schenkel 121, 122 wieder in die in Fig. 17 mit strichtpunktierten Linien angedeutete Öffnungsstellung schwenken, und zwar in Richtung der Pfeile 166, 167. Die Pinzette 32 schließt wieder und ihre Schenkel 33, 34 schwenken wieder in die in Fig. 17 mit strichpunktierten Linien angedeutete Schließstellung, bevor die Bogennadel 1 beim anschließenden Schwingen aus der in Fig. 17 mit ausgezogenen Linien wiedergegebenen Umkehrstellung in Richtung des Pfeils 25 in die Geweberänder einsticht, um dann bis in die in Fig. 17 mit strichpunktierten Linien angedeutete Umkehrstellung zu laufen.

Die Ausgangsstellung der Vorschubzange 120 und somit die Länge ihres Vor-und Rücklaufs zwischen der Ausgangsstellung und der Endstellung, also die Stichlänge der chirurgischen Nähmaschine, ist veränder- und einstellbar. Dazu ist die Steuerschaltung des umsteuerbaren elektrischen Schrittmotors 126 so ausgebildet, daß die Anzahl von Schritten verändert und eingestellt werden kann, welche der elektrische Schrittmotor 126 sowohl in der einen Richtung als auch in der anderen Richtung je Vorschub- und Rückbewegung der Zangenschenkel 121, 122 ausführt.

Die beiden Zangenschenkel 121, 122 sind an den freien Enden jeweils mit einer plattenförmigen Zunge 168 zur Anlage am benachbarten Geweberand versehen. Die beiden Zungen 168 sind auf den einander zugewandten Seiten 169 jeweils so ausgebildet, daß in der Schließstellung der Zangenschenkel 121, 122 jeglicher Schlupf zwischen den erfaßten Geweberändern und den Zungen 168 im wesentlichen ausgeschlossen ist. Beispielsweise können die Zungen 168 an den Seiten 169 mit Spitzen versehen oder sonstwie profiliert sein, um einen entsprechenden Formschluß mit den Geweberändern zu erzielen. Auch kann durch Beschichtung mit geeignetem Material für einen entsprechenden Reibschluß mit den Geweberändern gesorgt werden. In der Endstellung der Zangenschenkel 121, 122 befinden sich ihre Zungen 168 genau unterhalb der Zungen 38 der Pinzettenschenkel 33, 34 und des stationären Mittelarms 35, wie aus Fig. 18 besonders deutlich hervorgeht.

Die Vorschubzange 120 wird zusammen mit der Pinzette 32 gelüftet, und zwar mit Hilfe der Stellwelle 61, welche zu diesem Zweck mit dem zweiten Betätigungshebel 157 des linken Zangenschenkels 122 zusammenwirkt, um die Zangenschenkel 121, 122 entgegen der Wirkung der Schraubendruckfeder 123 auseinanderzuspreizen. Die Stellwelle 61 ist hohl ausgebildet, umschließt die Spindel 130 des rechten Zangenschenkels 121 und weist zusätzlich zur Nase 63 und zum ersten Arm 64 einen zweiten Arm 170 auf, welcher ebenfalls im wesentlichen radial absteht und über eine Drucklasche 171 mit dem steuernockenseitigen Arm 160 des zweiten Betätigungshebels 157 zusammenwirkt. Die am zweiten Arm 170 angelenkte Drucklasche 171 ist am anderen Ende mit einem Langloch 172 versehen, in welches ein Stift 173 am Arm 160 des zweiten Betätigungshebels 157 eingreift. Das Langloch 172 ermöglicht das Hin- und Herschwenken des zweiten Betätigungshebels 157 beim Umlauf des zugehörigen Steuernockens 124 mit der Steuerwalze 8 in Richtung des Pfeils 55 ohne Rückwirkung auf die Stellwelle 61, wenn diese bzw. deren Handhebel 62 sich in der Ruhestellung befindet.

Zu Sterilisationszwecken ist jeder Pinzettenschenkel 33 bzw. 34, der stationäre Mittelarm 35, jeder Zangenschenkel 121 bzw. 122, der Nadelhebel 4 und der Schwinggreifer 2 leicht lösbar an der Außenwelle 36 bzw. an der Innenwelle 37 bzw. an der Maschinengehäusevorderwand 31 bzw. an der rechten Spindel 130 bzw. an der linken Spindel 131 bzw. an der Nadelwelle 5 bzw. an der Greiferstange 6 befestigt, und zwar mittels einer bajonettverschlußartigen Schnellkupplung 174 bzw. 175 bzw. 176 bzw. 177 bzw. 178 bzw. 179 bzw. 180 mit einer Schraubendruckfeder 181.

Die Schnellkupplungen 177, 178 für die beiden Zangenschenkel 121, 122 sind jeweils von einem Kupplungszapfen 182 bzw. 183 des einen bzw. des anderen Zangenschenkels 121 bzw. 122 und von einem vorderen hülsenförmigen Abschnitt 130a bzw. 131a größeren Durchmessers der zugehörigen Spindel 130 bzw. 131 gebildet, welcher den Kupplungszapfen 182 bzw. 183 aufnimmt, in welchem die zugehörige Schraubendruckfeder 181 angeordnet ist und welcher in einer verhältnismäßig langen Bohrung 184 bzw. 185 entsprechenden Durchmessers in der Maschinengehäusevorderwand 31 drehbar und axial verschieblich gelagert ist. Jeder Spindelabschnitt 130a bzw. 131a ist von der zugehörigen Schwenkhülse 145 bzw. 146 umschlossen, welche im Maschinengehäuse 24 drehbar, aber axial unbeweglich gelagert ist.

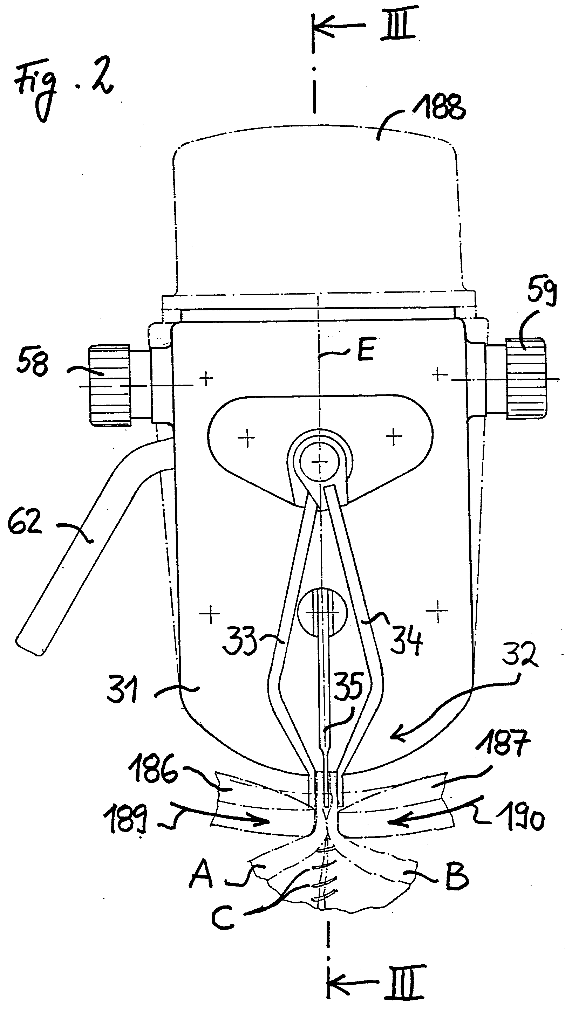

Bei der chirurgischen Nähmaschine nach Fig. 1 bis 8 sind für den schrittweisen Vorschub der Geweberänder A, B in Richtung des Pfeils 29 statt der Vorschubzange 120 zwei kreisrunde Vorschubscheiben 186, 187 vorgesehen, welche analog den Spindeln 130, 131 von einem auf dem Maschinengehäusedeckel 127 angeordneten elektrischen Schrittmotor 188 über ein Winkelgetriebe und ein Verteilergetriebe angetrieben werden, um sich intermittierend in Richtung der Pfeile 189, 190 in Fig. 2 zu drehen. Der elektrische Schrittmotor 188 braucht nicht umsteuerbar zu sein. Das Verteilergetriebe wirkt unmittelbar mit zwei Wellen zusammen, um sie in einander entgegengesetzten Richtungen zu drehen. Die Wellen erstrecken sich im Maschinengehäuse 24 analog den Spindeln 130, 131 und wirken jeweils über ein Winkelgetriebe 191 mit der rechten Vorschubscheibe 186 bzw. mit der linken Vorschubscheibe 187 zusammen. Analog den Zangenschenkeln 121, 122 sind die Vorschubscheiben 186, 187 aufeinander zu federbelastet und können sie entgegen der Wirkung der Federbelastung gelüftet werden, wozu jedes Winkelgetriebe 191 bzw. dessen Gehäuse in der Maschinengehäusevorderwand 31 drehbar gelagert ist. Auch ist jedes Winkelgetriebe 191 eingangsseitig mittels einer bajonettverschlußartigen Schnellkupplung leicht lösbar mit der zugehörigen Antriebswelle im Maschinengehäuse 24 verbunden, um die Vorschubscheiben 186, 187 mit dem jeweiligen Winkelgetriebe 191 zu Sterilisationszwecken abnehmen zu können.

## Patentansprüche

1. Chirurgische Nähmaschine zum Vernähen von Geweberändern (A, B) miteinander, welche
a) eine oszillierend angetriebene Nadel (1) und einen mit der Nadel (1) zur Stichbildung zusammenwirkenden Schlingenfänger (2), insbesondere eine Bogennadel und einen gabelförmigen Schwinggreifer, und
b) eine Vorrichtung zur Abstützung der Geweberänder (A, B) im unmittelbaren Bereich der sich quer durch die Geweberänder (A, B) erstreckenden Bewegungsbahn der Nadel (1)
aufweist, dadurch **gekennzeichnet**, daß als Geweberandabstützvorrichtung eine im Takt der Bewegungen der Nadel (1) schließende und öffnende Pinzette (32) mit zwei Schenkeln (33, 34) vorgesehen ist, welche vor jedem Einstechen der Nadel (1) in die Geweberänder (A, B) in eine Schließstellung zum Zusammenhalten der Geweberänder (A, B) und nach dem anschließenden Austreten der Nadel (1) aus den Geweberändern (A, B) in eine Öffnungsstellung zur Freigabe der Geweberänder (A, B) bewegbar sind.

2. Chirurgische Nähmaschine nach Anspruch 1, dadurch **gekennzeichnet**, daß die beiden Pinzettenschenkel (33, 34) durch mindestens eine Feder (40; 70, 71) in die Schließstellung belastet und durch mindestens einen Steuernocken (72; 41, 42) entgegen der Wirkung der Federbelastung in die Öffnungsstellung bewegbar sind.

3. Chirurgische Nähmaschine nach Anspruch 2, dadurch **gekennzeichnet**, daß die beiden Pinzettenschenkel (33, 34) durch eine gemeinsame Feder (40) in die Schließstellung belastet sind.

4. Chirurgische Nähmaschine nach Anspruch 3, dadurch **gekennzeichnet**, daß die Vorspannung der Feder (40) einstellbar ist.

5. Chirurgische Nähmaschine nach Anspruch 3 oder 4, dadurch **gekennzeichnet**, daß die Feder (40) als Schraubendruckfeder ausgebildet ist.

6. Chirurgische Nähmaschine nach Anspruch 2, dadurch **gekennzeichnet**, daß die beiden Pinzettenschenkel (33, 34) durch je eine Feder (70 bzw. 71) in die Schließstellung belastet sind.

7. Chirurgische Nähmaschine nach Anspruch 6, dadurch **gekennzeichnet**, daß die Vorspannung jeder Feder (70 bzw. 71) einstellbar ist.

8. Chirurgische Nähmaschine nach Anspruch 6 oder 7, dadurch **gekennzeichnet**, daß jede Feder (70 bzw. 71) als Schraubendruckfeder ausgebildet ist.

9. Chirurgische Nähmaschine nach einem der Ansprüche 2 bis 8, dadurch **gekennzeichnet**, daß die beiden Pinzettenschenkel (33, 34) durch je einen Steuernocken (41 bzw. 42) in die Öffnungsstellung bewegbar sind.

10. Chirurgische Nähmaschine nach Anspruch 9, dadurch **gekennzeichnet**, daß die beiden Steuernocken (41, 42) an einer Steuerwalze (8) für den Antrieb der Nadel (1) und des Schlingenfängers (2) vorgesehen sind.

11. Chirurgische Nähmaschine nach einem der Ansprüche 2 bis 8, dadurch **gekennzeichnet**, daß die beiden Pinzettenschenkel (33, 34) durch ein Getriebe (73) miteinander gekoppelt und durch einen Steuernocken (72) in die Öffnungsstellung bewegbar sind.

12. Chirurgische Nähmaschine nach Anspruch 11, dadurch **gekennzeichnet**, daß der Steuernocken (72) an einer Steuerwalze (8) für den Antrieb der Nadel (1) und des Schlingenfängers (2) vorgesehen ist.

13. Chirurgische Nähmaschine nach einem der vorstehenden Ansprüche, dadurch **gekennzeichnet**, daß die Schließstellungsposition (Sᵣ bzw. Sₗ) jedes Pinzettenschenkels (33 bzw. 34) einstellbar ist.

14. Chirurgische Nähmaschine nach Anspruch 13 in Verbindung mit einem der Ansprüche 2 bis 12, dadurch **gekennzeichnet**, daß jedem Pinzettenschenkel (33 bzw. 34) ein federseitiger erster Betätigungshebel (43 bzw. 44; 74 bzw. 75) und ein steuernockenseitiger zweiter Betätigungshebel (45 bzw. 46; 76 bzw. 77) zugeordnet sind, welche über ein Einstellglied (53 bzw. 54; 80 bzw. 81) zusammenwirken.

15. Chirurgische Nähmaschine nach Anspruch 14, wobei die beiden Pinzettenschenkel durch eine gemeinsame Schraubendruckfeder in die Schließstellung belastet sind, dadurch **gekennzeichnet**, daß die Schraubendruckfeder (40) zwischen den beiden ersten Betätigungshebeln (43, 44) angeordnet ist, welche sich innerhalb der beiden zweiten Betätigungshebel (45, 46) erstrecken, und daß die beiden Einstellglieder (53, 54) als Druckstifte zur äußeren Anlage an den ersten Betätigungshebeln (43, 44) ausgebildet sind, welche in den zweiten Betätigungshebeln (45, 46) verschraubbar sind.

16. Chirurgische Nähmaschine nach Anspruch 14, wobei die beiden Pinzettenschenkel durch je eine Schraubendruckfeder in die Schließstellung belastet sind, dadurch **gekennzeichnet**, daß die beiden Schraubendruckfedern (70, 71) außerhalb der beiden ersten Betätigungshebel (74, 75) angeordnet sind, innerhalb welcher sich die beiden zweiten Betätigungshebel (76, 77) erstrecken, und daß die beiden Einstellglieder (80, 81) als Druckstifte zur äußeren Anlage an den zweiten Betätigungshebeln (76, 77) ausgebildet sind, welche in den ersten Betätigungshebeln (74, 75) verschraubbar sind.

17. Chirurgische Nähmaschine nach Anspruch 14, 15 oder 16, wobei die beiden Pinzettenschenkel durch je einen Steuernocken in die Öffnungsstellung bewegbar sind, dadurch **gekennzeichnet**, daß jeder zweite Betätigungshebel (45 bzw. 46) einen Arm (51 bzw. 52) für das Zusammenwirken mit dem zugehörigen ersten Betätigungshebel (43 bzw. 44) und einen Arm (47 bzw. 48) für das Zusammenwirken mit dem zugehörigen Steuernocken (41 bzw. 42) aufweist.

18. Chirurgische Nähmaschine nach Anspruch 14, 15 oder 16, wobei die beiden Pinzettenschenkel durch ein Getriebe miteinander gekoppelt und durch einen Steuernocken in die Öffnungsstellung bewegbar sind, dadurch **gekennzeichnet**, daß jeder zweite Betätigungshebel (76 bzw. 77) einen Arm (78 bzw. 79) für das Zusammenwirken mit dem zugehörigen ersten Betätigungshebel (74 bzw. 75) und einen getriebeseitigen Arm (83 bzw. 85) aufweist, wobei ein zweiter Betätigungshebel (76) mit einem dritten Arm (82) für das Zusammenwirken mit dem Steuernocken (72) versehen ist.

19. Chirurgische Nähmaschine nach einem der Ansprüche 14 bis 18, **gekennzeichnet** durch einen Anschlag (105) zwischen dem ersten Betätigungshebel (74) des einen Pinzettenschenkels (33) und dem zweiten Betätigungshebel (77) des anderen Pinzettenschenkels (34) zur Festlegung des gegenseitigen Abstandes dieser Betätigungshebel (74, 77) in der Schließstellung der beiden Pinzettenschenkel (33, 34) und/oder einen Anschlag (104) zwischen dem ersten Betätigungshebel (75) des anderen Pinzettenschenkels (34) und dem zweiten Betätigungshebel (76) des einen Pinzettenschenkels (33) zur Festlegung des gegenseitigen Abstandes dieser Betätigungshebel (75, 76) in der Schließstellung der beiden Pinzettenschenkel (33, 34).

20. Chirurgische Nähmaschine nach Anspruch 19 in Verbindung mit Anspruch 18, **gekennzeichnet** durch eine solche Ausbildung des mit den beiden zweiten Betätigungshebeln (76, 77) zusammenwirkenden Getriebes (73), daß diese Betätigungshebel (76, 77) bezüglich einer maschinengehäusefesten Symmetrieebene (E) identische Bewegungen in einander entgegengesetzten Richtungen ausführen.

21. Chirurgische Nähmaschine nach einem der vorstehenden Ansprüche, **gekennzeichnet** durch eine hohle Außenwelle (36) und eine in derselben aufgenommene Innenwelle (37), welche gegenseitig verschwenkbar und jeweils mit dem einen bzw. dem anderen im wesentlichen radial abstehenden Pinzettenschenkel (33 bzw. 34) versehen sind.

22. Chirurgische Nähmaschine nach Anspruch 21 in Verbindung mit einem der Ansprüche 14 bis 20, dadurch **gekennzeichnet**, daß die beiden Pinzettenschenkel (33, 34) jeweils am einen Ende der Außenwelle (36) bzw. der Innenwelle (37) angeordnet sind, an deren anderem Ende der dem einen Pinzettenschenkel (33) zugeordnete erste Betätigungshebel (43; 74) bzw. der dem anderen Pinzettenschenkel (34) zugeordnete erste Betätigungshebel (44; 75) im wesentlichen radial absteht.

23. Chirurgische Nähmaschine nach Anspruch 22, dadurch **gekennzeichnet**, daß der zweite Betätigungshebel (45; 76) des einen Pinzettenschenkels (33) auf der Außenwelle (36) und der zweite Betätigungshebel (46; 77) des anderen Pinzettenschenkels (34) auf der Innenwelle (37) drehbar gelagert ist.

24. Chirurgische Nähmaschine nach Anspruch 23, **gekennzeichnet** durch eine zur Außenwelle (36) und Innenwelle (37) parallele Stellwelle (61; 108) für das Pinzettenlüften, welche mit mindestens einem der beiden zweiten Betätigungshebel (45, 46; 76, 77) zusammenwirkt und zum Auseinanderspreizen der beiden Pinzettenschenkel (33, 34) entgegen der Wirkung ihrer Federbelastung mittels eines Handhebels (62; 110) drehbar ist.

25. Chirurgische Nähmaschine nach Anspruch 24, wobei die beiden Pinzettenschenkel durch je einen Steuernocken in die Öffnungsstellung bewegbar sind, dadurch **gekennzeichnet**, daß die Stellwelle (61) eine im wesentlichen radial abstehende Nase (63) zum Verschwenken des zweiten Betätigungshebels (45) des einen Pinzettenschenkels (33) und einen im wesentlichen radial abstehenden Arm (64) zum Verschwenken des zweiten Betätigungshebels (46) des anderen Pinzettenschenkels (34) aufweist, wobei der Arm (64) durch eine Drucklasche (65) mit dem zweiten Betätigungshebel (46) des anderen Pinzettenschenkels (34) verbunden ist.

26. Chirurgische Nähmaschine nach Anspruch 24, wobei die beiden Pinzettenschenkel durch ein Getriebe miteinander gekoppelt und durch einen Steuernocken in die Öffnungsstellung bewegbar sind, dadurch **gekennzeichnet**, daß die Stellwelle (108) eine im wesentlichen radial abstehende Nase (109) zum Verschwenken des zweiten Betätigungshebels (76) eines Pinzettenschenkels (33) aufweist.

27. Chirurgische Nähmaschine nach Anspruch 23, 24 oder 26 in Verbindung mit Anspruch 20, dadurch **gekennzeichnet**, daß das Getriebe (73) als Gestänge ausgebildet ist und die beiden getriebeseitigen Arme (83, 85) der beiden zweiten Betätigungshebel (76, 77) über je eine Verbindungslasche (86 bzw. 87) an das eine Ende eines Schwenkhebels (88) angelenkt sind, welcher am anderen Ende um eine zur Außenwelle (36) und Innenwelle (37) parallele Achse (89) am Maschinengehäuse (24) verschwenkbar ist.

28. Chirurgische Nähmaschine nach einem der Ansprüche 21 bis 27, dadurch **gekennzeichnet**, daß die Innenwelle (37) hohl ausgebildet ist und eine hin- und herschwenkbare Nadelwelle (5) sich durch die Innenwelle (37) erstreckt.

29. Chirurgische Nähmaschine nach einem der Ansprüche 21 bis 28, dadurch **gekennzeichnet**, daß die beiden Pinzettenschenkel (33, 34) jeweils mittels einer Schnellkupplung (174 bzw. 175) an der Außenwelle (36) bzw. an der Innenwelle (37) befestigt sind.

30. Chirurgische Nähmaschine nach einem der vorstehenden Ansprüche, dadurch **gekennzeichnet**, daß jeder Pinzettenschenkel (33 bzw. 34) am freien Ende eine plattenförmige Zunge (38) zur Anlage am benachbarten Geweberand (A bzw. B) aufweist, welche mit einer Durchgangsöffnung (39) für die Nadel (1) versehen ist.

31. Chirurgische Nähmaschine nach einem der vorstehenden Ansprüche, **gekennzeichnet** durch einen stationären Mittelarm (35), mit welchem die beiden Pinzettenschenkel (33, 34) in der Schließstellung zusammenwirken, um die Geweberänder (A, B) zusammenzuhalten.

32. Chirurgische Nähmaschine nach Anspruch 31, dadurch **gekennzeichnet**, daß der stationäre Mittelarm (35) mittels einer Schnellkupplung (176) an der Maschinengehäusevorderwand (31) befestigt ist.

33. Chirurgische Nähmaschine nach Anspruch 31 oder 32, dadurch **gekennzeichnet**, daß der stationäre Mittelarm (35) am freien Ende eine plattenförmige Zunge (38) zur Anlage an den benachbarten Geweberändern (A, B) aufweist, welche mit einer Durchgangsöffnung (39) für die Nadel (1) versehen ist.

34. Chirurgische Nähmaschine nach einem der vorstehenden Ansprüche, **gekennzeichnet** durch eine im Takt des Öffnens und Schließens der Pinzette (32) arbeitende Vorschubzange (120) mit zwei Schenkeln (121, 122), welche
a) in eine Schließstellung zum Erfassen der Geweberänder (A, B) unmittelbar jenseits der Pinzette (32) und in eine Öffnungsstellung zur Freigabe der Geweberänder (A, B) sowie
b) während jeder Öffnungsperiode der Pinzette (32) in der Schließstellung aus einer Ausgangsstellung in Vorschubrichtung (29) und während jeder Schließperiode der Pinzette (32) in der Öffnungsstellung entgegen der Vorschubrichtung zurück in die Ausgangsstellung bewegbar sind.

35. Chirurgische Nähmaschine nach Anspruch 34, dadurch **gekennzeichnet**, daß die beiden Zangenschenkel (121, 122) durch mindestens eine Feder (123) in die Schließstellung belastet und durch mindestens einen Steuernocken (124) entgegen der Wirkung der Federbelastung in die Öffnungsstellung bewegbar sind.

36. Chirurgische Nähmaschine nach Anspruch 35, dadurch **gekennzeichnet**, daß die beiden Zangenschenkel (121, 122) durch eine gemeinsame Feder (123) in die Schließstellung belastet sind.

37. Chirurgische Nähmaschine nach Anspruch 36, dadurch **gekennzeichnet**, daß die Vorspannung der Feder (123) einstellbar ist.

38. Chirurgische Nähmaschine nach Anspruch 36 oder 37, dadurch **gekennzeichnet**, daß die Feder (123) als Schraubendruckfeder ausgebildet ist.

39. Chirurgische Nähmaschine nach einem der Ansprüche 35 bis 38, dadurch **gekennzeichnet**, daß die beiden Zangenschenkel (121, 122) durch ein Getriebe (125) miteinander gekoppelt und durch einen Steuernocken (124) in die Öffnungsstellung bewegbar sind.

40. Chirurgische Nähmaschine nach Anspruch 39, dadurch **gekennzeichnet**, daß der Steuernocken (124) an einer bzw. an der Steuerwalze (8) für den Antrieb der Nadel (1) und des Schlingenfängers (2) vorgesehen ist.

41. Chirurgische Nähmaschine nach einem der Ansprüche 34 bis 40, dadurch **gekennzeichnet**, daß die Schließstellungsposition (S'ᵣ bzw. S'ₗ) jedes Zangenschenkels (121 bzw. 122) einstellbar ist.

42. Chirurgische Nähmaschine nach Anspruch 41 in Verbindung mit Anspruch 39 oder 40, dadurch **gekennzeichnet**, daß dem einen Zangenschenkel (121) nur ein feder- und getriebeseitiger Betätigungshebel (151) zugeordnet ist, während dem anderen Zangenschenkel (122) ein feder- und getriebeseitiger erster Betätigungshebel (152) sowie ein steuernockenseitiger zweiter Betätigungshebel (157) zugeordnet sind, welche über ein Einstellglied (162) zusammenwirken.

43. Chirurgische Nähmaschine nach Anspruch 42, wobei die beiden Zangenschenkel durch eine gemeinsame Schraubendruckfeder in die Schließstellung belastet sind, dadurch **gekennzeichnet**, daß der zweite Betätigungshebel (157) auf der der Schraubendruckfeder (123) abgewandten Seite des ersten Betätigungshebels (152) angeordnet und das Einstellglied (162) als Druckstift zur äußeren Anlage am ersten Betätigungshebel (152) ausgebildet ist, welcher im zweiten Betätigungshebel (157) verschraubbar ist.

44. Chirurgische Nähmaschine nach einem der Ansprüche 34 bis 43, dadurch **gekennzeichnet**, daß die beiden Zangenschenkel (121,122) durch einen umsteuerbaren elektrischen Schrittmotor (126) in Vorschubrichtung (29) und entgegen der Vorschubrichtung bewegbar sind.

45. Chirurgische Nähmaschine nach Anspruch 44, dadurch **gekennzeichnet**, daß die Anzahl der Schritte einstellbar ist, welche der elektrische Schrittmotor (126) sowohl in der einen Richtung je Vorschubbewegung der beiden Zangenschenkel (121, 122) als auch in der anderen Richtung je Rückbewegung der beiden Zangenschenkel (121, 122) macht.

46. Chirurgische Nähmaschine nach Anspruch 44 oder 45, dadurch **gekennzeichnet**, daß jedem Zangenschenkel (121 bzw. 122) eine Spindel (130 bzw. 131) zugeordnet ist, welche mittels eines vom elektrischen Schrittmotor (126) hin- und herdrehbaren Ringes (141 bzw. 142) axial hinund herschiebbar ist, der mit einem Innengewinde (143 bzw. 144) auf einem entsprechenden Außengewinde (139 bzw. 140) der Spindel (130 bzw. 131) verschraubbar ist.

47. Chirurgische Nähmaschine nach Anspruch 46, dadurch **gekennzeichnet**, daß den beiden Ringen (141, 142) ein Verteilergetriebe (129) mit einem vom elektrischen Schrittmotor (126) angetriebenen Eingangszahnrad (136) und zwei Ausgangszahnrädern (137, 138) vorgeschaltet ist, welche jeweils mit dem Eingangszahnrad (136) kämmen und mit dem einen Ring (141) bzw. mit dem anderen Ring (142) verbunden sind.

48. Chirurgische Nähmaschine nach Anspruch 47, dadurch **gekennzeichnet**, daß jeder Ring (141 bzw. 142) und das zugehörige Ausgangszahnrad (137 bzw. 138) koaxial zueinander angeordnet und einstückig ausgebildet sind.

49. Chirurgische Nähmaschine nach Anspruch 47 oder 48, dadurch **gekennzeichnet**, daß zwischen dem elektrischen Schrittmotor (126) und dem Verteilergetriebe (129) ein Winkelgetriebe (128) vorgesehen ist.

50. Chirurgische Nähmaschine nach Anspruch 49, dadurch **gekennzeichnet**, daß das Winkelgetriebe (128) aus zwei miteinander kämmenden Kegelrädern (132, 133) besteht, welche jeweils mit der Ausgangswelle (134) des elektrischen Schrittmotors (126) bzw. mit der Eingangswelle (135) des Verteilergetriebes (129) verbunden sind.

51. Chirurgische Nähmaschine nach einem der Ansprüche 46 bis 50, dadurch **gekennzeichnet**, daß die beiden Spindeln (130, 131) mittels zweier hin- und herdrehbarer Schwenkhülsen (145, 146) zur Bewegung der beiden Zangenschenkel (121, 122) in die Schließstellung und in die Öffnungsstellung hin- und herschwenkbar sind, wobei die beiden Spindeln (130, 131) jeweils in der einen Schwenkhülse (145) bzw. in der anderen Schwenkhülse (146) bei drehfester Verbindung mit derselben axial verschieblich sind.

52. Chirurgische Nähmaschine nach Anspruch 51 in Verbindung mit Anspruch 42 oder 43, dadurch **gekennzeichnet**, daß die beiden Schwenkhülsen (145, 146) jeweils mit dem dem einen Zangenschenkel (121) zugeordneten bzw. mit dem dem anderen Zangenschenkel (122) zugeordneten und im wesentlichen radial abstehenden feder- und getriebeseitigen Betätigungshebel (151 bzw. 152) versehen sind.

53. Chirurgische Nähmaschine nach Anspruch 52, dadurch **gekennzeichnet**, daß das die beiden Zangenschenkel (121, 122) miteinander koppelnde Getriebe (125) als Gestänge mit zwei Armen (153, 154) ausgebildet ist, welche jeweils von der einen Schwenkhülse (145) bzw. von der anderen Schwenkhülse (146) im wesentlichen radial abstehen und welche an den freien Enden mittels eines Querstiftes (155) und einer langlochartigen Ausnehmung (156) zur Aufnahme des Querstiftes (155) ineinandergreifen.

54. Chirurgische Nähmaschine nach Anspruch 52 oder 53, dadurch **gekennzeichnet**, daß der zweite Betätigungshebel (157) des einen Zangenschenkels (122) zwei Arme (159, 160) zum Zusammenwirken mit dem ersten Betätigungshebel (152) des Zangenschenkels (122) bzw. zum Zusammenwirken mit dem Steuernocken (124) aufweist, welche im wesentlichen radial von einer rohrförmigen Nabe (158) abstehen, die auf derselben Spindel (131) angeordnet ist, welche sich auch durch die mit dem ersten Betätigungshebel (152) versehene Schwenkhülse (146) erstreckt.

55. Chirurgische Nähmaschine nach einem der Ansprüche 46 bis 54, wobei die beiden Pinzettenschenkel jeweils an einer hohlen Außenwelle bzw. an einer in derselben aufgenommenen Innenwelle vorgesehen sind, dadurch **gekennzeichnet**, daß die beiden Spindeln (130, 131) parallel zur Außenwelle (36) und Innenwelle (37) angeordnet sind und die beiden Zangenschenkel (121, 122) jeweils im wesentlichen radial von der einen Spindel (130) bzw. von der anderen Spindel (131) abstehen.

56. Chirurgische Nähmaschine nach Anspruch 55 in Verbindung mit Anspruch 54, wobei eine zur Außenwelle und Innenwelle parallele Stellwelle für das Pinzettenlüften vorgesehen ist, welche zum Auseinanderspreizen der beiden Pinzettenschenkel entgegen der Wirkung ihrer Federbelastung mittels eines Handhebels drehbar ist, dadurch **gekennzeichnet**, daß die Stellwelle (61) hohl ausgebildet, auf der zu der sich durch die Schwenkhülse (146) mit dem ersten Betätigungshebel (152) und durch die rohrförmige Nabe (158) des zweiten Betätigungshebels (157) erstreckenden Spindel (131) parallelen Spindel (130) angeordnet und zum Vorschubzangenlüften mit einem im wesentlichen radial abstehenden Arm (170) versehen ist, welcher durch eine Drucklasche (171) mit dem zweiten Betätigungshebel (157) verbunden ist, um die beiden Zangenschenkel (121, 122) beim Drehen der Stellwelle (61) mittels des Handhebels (62) entgegen der Wirkung ihrer Federbelastung auseinanderzuspreizen.

57. Chirurgische Nähmaschine nach einem der Ansprüche 46 bis 56, dadurch **gekennzeichnet**, daß die beiden Zangenschenkel (121, 122) jeweils mittels einer Schnellkupplung (177 bzw. 178) an der einen Spindel (130) bzw. an der anderen Spindel (131) befestigt sind.

58. Chirurgische Nähmaschine nach einem der Ansprüche 34 bis 57, dadurch **gekennzeichnet**, daß die beiden Zangenschenkel (121, 122) an den freien Enden jeweils eine plattenförmige Zunge (168) zur Anlage am benachbarten Geweberand aufweisen und die beiden Zungen (168) auf den einander zugewandten Seiten (169) jeweils so ausgebildet sind, daß in der Schließstellung der beiden Zangenschenkel (121, 122) jeglicher Schlupf zwischen den erfaßten Geweberändern und den beiden Zungen (121, 122) im wesentlichen ausgeschlossen ist.

## Claims

1. Surgical sewing machine for suturing edges (A, B) of tissue together comprising
a) an oscillatingly driven needle (1) and a loop catcher (2) co-operating with the needle (1) to form the stitches, in particular a curved needle and a forked swinging gripper, and
b) a device for supporting the edges (A, B) of the tissue in the immediate region of the path of movement of the needle (1) which extends transverse to the edges (A, B) of the tissue,
**characterised in that** as the tissue-edge-engaging device there are provided forceps (32) having two legs (33, 34) opening and closing in synchronism with the movements of the needle and which before each insertion of the needle (1) in the edges (A, B) of the tissue are moved into a closed position for holding together the edges (A, B) of the tissue and after the subsequent emergence of the needle (1) from the edges (A, B) of the tissue they are moved into an open position to release the edges (A, B) of the tissue.

2. Surgical sewing machine according to claim 1, **characterised in that** the two legs (33, 34) of the forceps are urged into the closed position by at least one spring (40; 70, 71) and are moved into the open position against the action of the spring loading by at least one control cam (72; 41, 42).

3. Surgical sewing machine according to claim 2, **characterised in that** the two legs (33, 34) of the forceps are urged into the closed position by a common spring (40).

4. Surgical sewing machine according to claim 3, **characterised in that** the loading of the spring (40) is adjustable.

5. Surgical sewing machine according to claim 3 or 4, **characterised in that** the spring (40) is in the form of a coil compression spring.

6. Surgical sewing machine according to claim 2, **characterised in that** the two legs (33, 34) of the forceps are each urged into the closed position by a respective spring (70 and 71).

7. Surgical sewing machine according to claim 6, **characterised in that** the loading of each spring (70 and 71) is adjustable.

8. Surgical sewing machine according to claim 6 or 7, **characterised in that** each spring (70 and 71) is in the form of a coil compression spring.

9. Surgical sewing machine according to one of claims 2 to 8, **characterised in that** the two legs (33, 34) of the forceps are each moved into the open position by a respective control cam (41 and 42).

10. Surgical sewing machine according to claim 9, **characterised in that** the two control cams (41, 42) are provided on a control roller (8) for driving the needle (1) and the loop catcher (2).

11. Surgical sewing machine according to one of claims 2 to 8, **characterised in that** the two legs (33, 34) of the forceps are coupled together by gearing (73) and are moved into the open position by a control cam (72).

12. Surgical sewing machine according to claim 11, **characterised in that** the control cam (72) is provided on a control roller (8) for driving the needle (1) and the loop catcher (2).

13. Surgical sewing machine according to one of the foregoing claims, **characterised in that** the closed position (Sᵣ and Sₗ) of each leg (33, 34) of the forceps is adjustable.

14. Surgical sewing machine according to claim 13 in conjunction with one of claims 2 to 12, **characterised in that** each leg (33 and 34) of the forceps has a spring-side first actuating lever (43 and 44; 74 and 75) and a control-cam-side second actuating lever (45 and 46; 76 and 77) associated with it and cooperating through an adjusting member (53 and 54; 80 and 81).

15. Surgical sewing machine according to claim 14, the two legs of the forceps being urged into the closed position by a common coil compression spring, **characterised in that** the coil compression spring (40) is mounted between the two first actuating levers (43, 44) which extend within the two second actuating levers (45, 46), and that the two adjusting members (53, 54) are in the form of pressure pins for external engagement against the first actuating levers (43, 44) and screwed into the second actuating levers (45, 46).

16. Surgical sewing machine according to claim 14, the two legs of the forceps each being urged into the closed position by a respective coil compression spring, **characterised in that** the two coil compression springs (70, 71) are arranged outside the two first actuating levers (74, 75), within which the two second actuating levers (76, 77) extend, and that the two adjusting members (80,81) are in the form of pressure pins for external engagement against the second actuating levers (76, 77) and screwed into the first actuating levers (74, 75).

17. Surgical sewing machine according to claim 14, 15 or 16, the two legs of the forceps each being moved into the open position by a respective control cam, **characterised in that** each second actuating lever (45 and 46) has an arm (51 and 52) for cooperating with the associated first actuating lever (43 and 44) and an arm (47 and 48) for co-operating with the associated control cam (41 and 42).

18. Surgical sewing machine according to claim 14, 15 or 16, the two legs of the forceps being coupled together by gearing and being moved into the open position by a control cam, **characterised in** **that** each second actuating lever (76 and 77) has an arm (78 and 79) for co-operating with the associated first actuating lever (74 and 75) and a gearing-side arm (83 and 85), one second actuating lever (76) being provided with a third arm (82) for co-operating with the control cam 72).

19. Surgical sewing machine according to one of claims 14 to 18, **characterised by** a stop (105) between the first actuating lever (74) of the one leg (33) of the forceps and the second actuating lever (77) of the other leg (34) of the forceps for determining the mutual spacing of these levers (74, 77) in the closed position of the two legs (33, 34) and/or a stop (104) between the first actuating lever (75) of the other leg (34) of the forceps and the second actuating lever (76) of the one leg (33) of the forceps for determining the mutual spacing of these levers (75, 76) in the closed position of the two legs (33, 34).

20. Surgical sewing machine according to claim 19 in conjunction with claim 18, **characterised by** such a construction of the gearing (73) co-operating with the two second actuating levers (76, 77) that these actuating levers (76, 77) perform identical movements in mutually opposite directions with reference to a plane of symmetry (E) fixed in relation to the housing of the machine.

21. Surgical sewing machine according to one of the foregoing claims, **characterised by** a hollow outer shaft (36) and an inner shaft (37) received in it, which can pivot mutually and each of which is provided with one or the other substantially radially projecting leg (33 and 34) of the forceps.

22. Surgical sewing machine according to claim 21 in conjunction with one of claims 14 to 20, **characterised in that** the two legs (33, 34) of the forceps are each arranged on one end of the outer shaft (36) and of the inner shaft (37) respectively, on the other ends of which there projects substantially radially the first actuating lever (43; 74) associated with the one leg (33) and the first actuating lever (44; 75) associated with the other leg (34).

23. Surgical sewing machine according to claim 22, **characterised in that** the second actuating lever (45; 76) of the one leg (33) of the forceps is rotatably mounted on the outer shaft (36) and the second actuating lever (46; 77) of the other leg (34) of the forceps is rotatably mounted on the inner shaft (37).

24. Surgical sewing machine according to claim 23, **characterised by** an actuating shaft (61; 108) parallel to the outer shaft (36) and inner shaft (37) for releasing the forceps and which co-operates with at least one of the two second actuating levers (45, 46; 76, 77) and is rotatable by means of a hand lever (62; 110) for spreading apart the two legs (33, 34) of the forceps against the action of their spring loading.

25. Surgical sewing machine according to claim 24, the two legs of the forceps each being moved into the open position by a respective control cam, **characterised in that** the actuating shaft (61) has a substantially radially projecting nose (63) for pivoting the second actuating lever (45) of the one leg (33) of the forceps and a substantially radially projecting arm (64) for pivoting the second actuating lever (46) of the other leg (34) of the forceps, the arm (64) being connected through a pressure link (65) to the second actuating lever (46) of the other leg (34) of the forceps.

26. Surgical sewing machine according to claim 24, the two legs of the forceps being coupled together by gearing and being moved by a control cam into the open position, **characterised in that** the actuating shaft (108) has a substantially radially projecting nose (109) for pivoting the second actuating lever (76) of one leg (33) of the forceps.

27. Surgical sewing machine according to claim 23, 24 or 26 in conjunction with claim 20, **characterised in that** the gearing (73) is in the form of a linkage and the two gearing-side arms (83, 85) of the two second actuating levers (76, 77) are each pivoted through a connecting link (86 and 87) to the one end of a swinging lever (88), which pivots at its other end on the housing (24) of the machine about an axis (89) parallel to the outer shaft (36) and the inner shaft (37).

28. Surgical sewing machine according to one of claims 21 to 27 **characterised in that** the inner shaft (37) is made hollow and a needle shaft (5) which can swing back and forth extends through the inner shaft (37).

29. Surgical sewing machine according to one of claims 21 to 28, **characterised in that** the two legs (33, 34) of the forceps are each secured by means of a quick-release coupling (174 and 175) to the outer shaft (36) and to the inner shaft (37) respectively.

30. Surgical sewing machine according to one of the foregoing claims, **characterised in that** each leg (33 and 34) of the forceps is provided on its free end with a plate-shaped tongue (38) for engaging the adjacent edge (A and B) of the tissue and provided with an opening (39) for the passage of the needle (1).

31. Surgical sewing machine according to one of the foregoing claims, **characterised by** a stationary central arm (35) with which the two legs (33, 34) of the forceps cooperate in the closed position in order to hold together the edges (A, B) of the tissue.

32. Surgical sewing machine according to claim 31, **characterised in that** the stationary central arm (35) is secured on the front wall (31) of the housing of the machine by means of a quick-release coupling (176).

33. Surgical sewing machine according to claim 31 or 32, **characterised in that** the stationary central arm (35) has on its free end a plate-shaped tongue (38) for engaging the adjacent edges (A, B) of the tissue and provided with an opening (39) for the passage of the needle (1).

34. Surgical sewing machine according to one of the foregoing claims, **characterised by** a feed claw (120) working in synchronism with the opening and closing of the forceps (32) and having two limbs (121, 122) which are moved
a) into a closed position for grasping the edges (A, B) of the tissue immediately beyond the forceps (32) and into an open position for releasing the edges (A, B) of the tissue as well as
b) being moved during each open period of the forceps (32) in the closed position from a starting position in the direction of feed (29) and during each closed period of the forceps (32) being moved in the open position in a direction opposite to the direction of feed and back to the starting position.

35. Surgical sewing machine according to claim 34, **characterised in that** the two limbs (121, 122) of the claw are urged into the closed position by at least one spring (123) and are moved into the open position against the action of the spring loading by at least one control cam (124).

36. Surgical sewing machine according to claim 35, **characterised in that** the two limbs (121, 122) of the claw are urged into the closed position by a common spring (123).

37. Surgical sewing machine according to claim 36, **characterised in that** the loading of the spring (123) is adjustable.

38. Surgical sewing machine according to claim 36, or 37, **characterised in that** the spring (123) is in the form of a coil compression spring.

39. Surgical sewing machine according to one of claims 35 to 38, **characterised in that** the two limbs (121, 122) of the claw are coupled together by gearing (125) and are moved into the open position by a control cam (124).

40. Surgical sewing machine according to claim 39, **characterised in that** the control cam (124) is provided on a or the control roller (8) for driving the needle (1) and the loop catcher (2).

41. Surgical sewing machine according to one of claims 34 to 40, **characterised in that** the closed position (S'ᵣ and S'ₗ) of each limb (121 and 122) of the claw is adjustable.

42. Surgical sewing machine according to claim 41 in conjunction with claim 39 or 40, **characterised in that** there is associated with the one limb (121) of the claw only one spring-side and gearing-side actuating lever (151), whilst the other limb (122) of the claw has associated with it a spring-side and gearing-side first actuating lever (152) as well as a control-cam-side second actuating lever (157) which co-operate through an adjusting member (162).

43. Surgical sewing machine according to claim 42, the two limbs of the claw being urged into the closed position by a common coil compression spring, **characterised in that** the second actuating lever (157) is arranged on that side of the first actuating lever (152) furthest from the coil compression spring (123) and that the adjusting member (162) is in the form of a pressure pin for external engagement against the first actuating lever (152) and screwed into the second actuating lever (157).

44. Surgical sewing machine according to one of claims 34 to 43, **characterised in that** the two limbs (121, 122) of the claw are moved in the feed direction (29) and in the opposite direction by a reversible electric stepping motor (126).

45. Surgical sewing machine according to claim 44, **characterised in that** the number of steps which the stepping motor (126) makes, both in the one direction for each feed movement of the two limbs (121, 122) of the claw and also in the opposite direction for each return movement of the two limbs (121, 122) is adjustable.

46. Surgical sewing machine according to claim 44 or 45, **characterised in that** each limb (121 and 122) of the claw has associated with it a spindle (130 and 131) which is displaceable axially back and forth by means of a ring (141 and 142) rotatable back and forth by the electric stepping motor (126), the ring being provided with an internal screw thread (143 and 144) screwed onto a corresponding external thread (139 and 140) on the spindle (130 and 131).

47. Surgical sewing machine according to claim 46, **characterised in that** the two rings (141, 142) have connected ahead of them a splitter box (129) with an input gear wheel (136) driven by the electric stepping motor (126) and two output gear wheels (137, 138) which each mesh with the input gear wheel (136) and are connected to the one ring (141) and to the other ring (142).

48. Surgical sewing machine according to claim 47, **characterised in that** each ring (141 and 142) and the associated output gear wheel (137 and 138) are arranged co-axially and made integrally.

49. Surgical sewing machine according to claim 47 or 48, **characterised in that** an angle gearing (128) is provided between the electric stepping motor (126) and the splitter box (129).

50. Surgical sewing machine according to claim 49, **characterised in that** the angle gearing (128) comprises two mutually meshing bevel wheels (132, 133), each of which is connected to the output shaft (134) of the electric stepping motor (126) and to the input shaft (135) of the splitter box (129) respectively.

51. Surgical sewing machine according to one of claims 46 to 50, **characterised in that** the two spindles (130, 131) are swung back and forth for moving the two limbs (121, 122) of the claw into the closed position and into the open position by means of two pivoting sleeves (145, 146) which are rotated back and forth, the two spindles (130, 131) each being axially displaceable in the one sleeve (145) and respectively in the other sleeve (146) whilst secured against rotation..

52. Surgical sewing machine according to claim 51 in conjunction with claim 42 or 43, **characterised in that** the two pivoting sleeves (145,146) are provided with the spring-side and gearing-side actuating levers (151 and 152) associated with the one limb (121) of the claw and with the other limb (122) of the claw and projecting substantially radially.

53. Surgical sewing machine according to claim 52, **characterised in that** the gearing (125) which couples the two limbs (121, 122) of the claw together is in the form of a linkage with two arms (153, 154) which each project substantially radially from the one pivoting sleeve (145) and from the other pivoting sleeve (146) respectively and which interengage at their free ends by means of a transverse pin (155) and a slot-like opening (156) for receiving the pin (155).

54. Surgical sewing machine according to claim 52 or 53, **characterised in that** the second actuating lever (157) of the one limb (122) of the claw has two arms (159, 160) for co-operating with the first actuating lever (152) of the limb (122) of the claw and for co-operating with the control cam (124), which project substantially radially from a tubular hub (158) which is arranged on the same spindle (131) which also extends through the pivoting sleeve (146) provided with the first actuating lever (152).

55. Surgical sewing machine according to one of claims 46 to 54, the two legs of the forceps each being provided on a hollow outer shaft and an inner shaft received within it respectively, **characterised in that** the two spindles (130, 131) are arranged parallel to the outer shaft (36) and inner shaft (37) and the two limbs (121, 122) of the claw each project substantially radially from the one spindle (130) and from the other spindle (131) respectively.

56. Surgical sewing machine according to claim 55 in conjunction with claim 54, an actuating shaft for releasing the forceps being provided parallel with the outer shaft and inner shaft and being rotatable by means of a hand lever for spreading apart the two legs of the forceps against the action of their spring loading, **characterised in that** the actuating shaft (61) is made hollow, arranged on the spindle (130) parallel to the spindle (131) extending through the pivoting sleeve (146) with the first actuating lever (152) and through the tubular hub (158) of the second actuating lever (157), and, for releasing the feed claw, provided with a substantially radially projecting arm (170) which is connected through a pressure link (171) to the second actuating lever (157), in order to spread apart the two limbs (121, 122) of the claw against the action of their spring loading on rotation of the actuating shaft (61) by means of the hand lever (62).

57. Surgical sewing machine according to one of claims 46 to 56, **characterised in that** the two limbs (121, 122) of the claw are each secured by means of a quick-release coupling (177 and (178) to the one spindle (130) and to the other spindle (131) respectively.

58. Surgical sewing machine according to one of claims 34 to 57, **characterised in that** the two limbs (121, 122) of the claw each have on the free end a respective plate-shaped tongue (168) for engaging the adjacent edge of the tissue and the two tongues (168) are each formed on their mutually facing sides (169) in such a way that in the closed position of the two limbs (121, 122) of the claw any slip between the grasped edges of the tissue and the two tongues (168) is substantially eliminated.

## Revendications

1. Machine à coudre chirurgicale pour coudre ensemble des bords de tissu (A, B), qui comporte
a) une aiguille (1) entraînée de manière à osciller et une navette circulaire (2) coopérant avec l'aiguille (1) pour former les points, notamment une aiguille courbe et une pince oscillante fourchue, et
b) un dispositif de maintien des bords de tissu (A, B) au voisinage immédiat du trajet de l'aiguille (1) s'étendant perpendiculairement à travers les bords de tissu (A,B),
caractérisée en ce qu'une pincette (32), qui se ferme et s'ouvre à la même cadence que les mouvements de l'aiguille (1) et qui comporte deux branches (33, 34), est prévue comme dispositif de maintien des bords de tissu, les branches de la pincette pouvant être mises en position fermée pour maintenir ensemble les bords de tissu (A, B) avant chaque piqûre de l'aiguille (1) dans les bords de tissu (A, B) et pouvant être mises en position ouverte pour libérer les bords de tissu (A, B) après chaque sortie consécutive de l'aiguille (1) hors des bords de tissu (A, B).

2. Machine à coudre chirurgicale selon la revendication 1, caractérisée en ce que les deux branches (33, 34) de la pincette sont contraintes en position fermée par au moins un ressort (40 ; 70, 71) et peuvent être mises en position ouverte contre l'action du ressort par au moins une came radiale (72 ; 41, 42).

3. Machine à coudre chirurgicale selon la revendication 2, caractérisée en ce que les deux branches (33, 34) de la pincette sont contraintes en position fermée par un ressort (40) commun.

4. Machine à coudre chirurgicale selon la revendication 3, caractérisée en ce que la précontrainte du ressort (40) est réglable.

5. Machine à coudre chirurgicale selon la revendication 3 ou 4, caractérisée en ce que le ressort (40) est construit comme un ressort hélicoïdal de compression.

6. Machine à coudre chirurgicale selon la revendication 2, caractérisée en ce que les deux branches (33, 34) de la pincette sont contraintes en position fermée chacune par un ressort (70 ou 71).

7. Machine à coudre chirurgicale selon la revendication 6, caractérisée en ce que la précontrainte de chaque ressort (70 ou 71) est réglable.

8. Machine à coudre chirurgicale selon la revendication 6 ou 7, caractérisée en ce que chaque ressort (70 ou 71) est construit comme un ressort hélicoïdal de compression.

9. Machine à coudre chirurgicale selon l'une des revendications 2 à 8, caractérisée en ce que les deux branches (33, 34) de la pincette peuvent être mises en position ouverte chacune par une came radiale (41 ou 42).

10. Machine à coudre chirurgicale selon la revendication 9, caractérisée en ce que les deux cames radiales (41, 42) sont prévues sur un cylindre de commande (8) destiné à l'entraînement de l'aiguille (1) et de la navette circulaire (2).

11. Machine à coudre chirurgicale selon l'une des revendications 2 à 8, caractérisée en ce que les deux branches (33, 34) de la pincette sont couplées ensemble par un mécanisme de transmission (73) et peuvent être mises en position ouverte par une came radiale (72).

12. Machine à coudre chirurgicale selon la revendication 11, caractérisée en ce que la came radiale (72) est prévue sur un cylindre de commande (8) destiné à l'entraînement de l'aiguille (1) et de la navette circulaire (2).

13. Machine à coudre chirurgicale selon l'une des revendications précédentes, caractérisée en ce que la position fermée (Sᵣ ou Sₗ) de chaque branche (33 ou 34) de la pincette est réglable.

14. Machine à coudre chirurgicale selon la revendication 13 et en relation avec l'une des revendications 2 à 12, caractérisée en ce qu'un premier levier d'actionnement (43 ou 44 ; 74 ou 75) situé du côté du ressort et un second levier d'actionnement (45 ou 46 ; 76 ou 77) situé du côté de la came radiale sont associés à chaque branche (33 ou 34) de la pincette et coopèrent par l'intermédiaire d'un élément de réglage (53 ou 54 ; 80 ou 81).

15. Machine à coudre chirurgicale selon la revendication 14, les deux branches de la pincette étant contraintes en position fermée par un ressort hélicoïdal de compression commun, caractérisée en ce que le ressort hélicoïdal de compression (40) est agencé entre les deux premiers leviers d'actionnement (43, 44) qui s'étendent à l'intérieur des deux seconds leviers d'actionnement (45, 46) et les deux éléments de réglage (53, 54) sont construits comme des tiges de poussée pour un appui de l'extérieur contre les premiers leviers d'actionnement (43, 44) et peuvent être vissés dans les seconds leviers d'actionnement (45, 46).

16. Machine à coudre chirurgicale selon la revendication 14, les deux branches de la pincette étant contraintes en position fermée chacune par un ressort hélicoïdal de compression, caractérisée en ce que les deux ressorts hélicoïdaux de compression (70, 71) sont agencés en dehors des deux premiers leviers d'actionnement (74, 75) à l'intérieur desquels les deux seconds leviers d'actionnement (76, 77) s'étendent et les deux éléments de réglage (80, 81) sont construits comme des tiges de poussée pour un appui de l'extérieur contre les seconds leviers d'actionnement (76, 77) et peuvent être vissés dans les premiers leviers d'actionnement (74, 75).

17. Machine à coudre chirurgicale selon la revendication 14, 15 ou 16, les deux branches de la pincette pouvant être mises en position ouverte chacune par une came radiale, caractérisée en ce que chaque second levier d'actionnement (45 ou 46) comporte un bras (51 ou 52) pour la coopération avec le premier levier d'actionnement (43 ou 44) associé et un bras (47 ou 48) pour la coopération avec la came radiale (41 ou 42) associée.

18. Machine à coudre chirurgicale selon la revendication 14, 15 ou 16, les deux branches de la pincette étant couplées ensemble par un mécanisme de transmission et pouvant être mises en position ouverte par une came radiale, caractérisée en ce que chaque second levier d'actionnement (76 ou 77) comporte un bras (78 ou 79) pour la coopération avec le premier levier d'actionnement (74 ou 75) associé et un bras (83 ou 85) situé du côté du mécanisme de transmission, un second levier d'actionnement (76) étant muni d'un troisième bras (82) pour la coopération avec la came radiale (72).

19. Machine à coudre chirurgicale selon l'une des revendications 14 à 18, caractérisée par une butée (105) située entre le premier levier d'actionnement (74) d'une branche (33) de la pincette et le second levier d'actionnement (77) de l'autre branche (34) de la pincette afin de fixer la distance entre ces leviers d'actionnement (74, 77) lorsque les deux branches (33, 34) de la pincette sont en position fermée, et/ou une butée (104) située entre le premier levier d'actionnement (75) de l'autre branche (34) de la pincette et le second levier d'actionnement (76) de la première branche (33) de la pincette afin de fixer la distance entre ces leviers d'actionnement (75, 76) lorsque les deux branches (33, 34) de la pincette sont en position fermée.

20. Machine à coudre chirurgicale selon la revendication 19 et en relation avec la revendication 18, caractérisée par une construction du mécanisme de transmission (73) coopérant avec les deux seconds leviers d'actionnement (76, 77) telle que ces leviers d'actionnement (76, 77) effectuent des déplacements identiques par rapport à un plan de symétrie (E) fixé par rapport au bâti de la machine mais dans des directions opposées l'une à l'autre.

21. Machine à coudre chirurgicale selon l'une des revendications précédentes, caractérisée par un arbre extérieur (36) creux et par un arbre intérieur (37) logé dans le précédent, qui peuvent pivoter l'un par rapport à l'autre et qui sont munis chacun de l'une ou l'autre des branches (33 ou 34) de la pincette qui s'éloignent globalement en direction radiale.

22. Machine à coudre chirurgicale selon la revendication 21 en relation avec l'une des revendications 14 à 20, caractérisée en ce que les deux branches (33, 34) de la pincette sont agencées respectivement à une extrémité de l'arbre extérieur (36) et à une extrémité de l'arbre intérieur (37), à l'autre extrémité desquels s'éloignent, globalement en direction radiale, respectivement le premier levier d'actionnement (43 ; 74) associé à la première branche (33) de la pincette et le premier levier d'actionnement (44 ; 75) associé à l'autre branche (34) de la pincette.

23. Machine à coudre chirurgicale selon la revendication 22, caractérisée en ce que le second levier d'actionnement (45 ; 76) de la première branche (33) de la pincette est monté de manière à pouvoir tourner sur l'arbre extérieur (36) et le second levier d'actionnement (46 ; 77) de l'autre branche (34) de la pincette est monté de manière à pouvoir tourner sur l'arbre intérieur (37).

24. Machine à coudre chirurgicale selon la revendication 23, caractérisée par un arbre de réglage (61 ; 108), parallèle à l'arbre extérieur (36) et à l'arbre intérieur (37) et destiné à desserrer la pincette, qui coopère avec au moins l'un des deux seconds leviers d'actionnement (45, 46 ; 76, 77) et qui peut être tourné au moyen d'un lever à main (62; 110) afin d'écarter les deux branches (33, 34) de la pincette contre l'action de leur contrainte par ressort.

25. Machine à coudre chirurgicale selon la revendication 24, les deux branches de la pincette pouvant être mises en position ouverte chacune par une came radiale, caractérisée en ce que l'arbre de réglage (61) comporte une came (63), qui s'étend globalement en direction radiale et qui sert à faire pivoter le second levier d'actionnement (45) de la première branche (33) de la pincette, et un bras (64), qui s'étend globalement en direction radiale et qui sert à faire pivoter le second levier d'actionnement (46) de l'autre branche (34) de la pincette, le bras (64) étant lié au second levier d'actionnement (46) de l'autre branche (34) de la pincette par une attache de poussée (65).

26. Machine à coudre chirurgicale selon la revendication 24, les deux branches de la pincette étant couplées ensemble par un mécanisme de transmission et pouvant être mises en position ouverte par une came radiale, caractérisée en ce que l'arbre de réglage (108) comporte une came (109) qui s étend globalement en direction radiale et qui sert à faire pivoter le second levier d'actionnement (76) d'une branche (33) de la pincette.

27. Machine à coudre chirurgicale selon la revendication 23, 24 ou 26 et en relation avec la revendication 20, caractérisée en ce que le mécanisme de transmission (73) est formé par des tiges et les deux bras (83, 85), situés du côté du mécanisme de transmission, des deux seconds leviers d'actionnement (76, 77) sont articulés sur une première extrémité d'un levier pivotant (88) chacun par l'intermédiaire d'une tige de liaison (86 ou 87), l'autre extrémité du levier pivotant pouvant pivoter autour d'un axe (89) parallèle à l'arbre extérieur (36) et à l'arbre intérieur (37) et monté sur le bâti (24) de la machine.

28. Machine à coudre chirurgicale selon l'une des revendications 21 à 27, caractérisée en ce que l'arbre intérieur (37) est creux et un arbre d'aiguille (5) pouvant aller et venir s'étend à travers l'arbre intérieur (37).

29. Machine à coudre chirurgicale selon l'une des revendications 21 à 28, caractérisée en ce que les deux branches (33, 34) de la pincette sont fixées respectivement à l'arbre extérieur (36) et à l'arbre intérieur (37) au moyen d'un raccord instantané (174 ou 175).

30. Machine à coudre chirurgicale selon l'une des revendications précédentes, caractérisée en ce que chaque branche (33 ou 34) de la pincette comporte sur son extrémité libre un ergot (38) en forme de plaque pour l'appui contre le bord de tissu (A ou B) adjacent, chaque ergot étant muni d'une ouverture de passage (39) pour l'aiguille (1).

31. Machine à coudre chirurgicale selon l'une des revendications précédentes, caractérisée par un bras central (35) immobile avec lequel les deux branches (33, 34) de la pincette coopèrent en position fermée pour maintenir rapprochés les bords de tissu (A, B).

32. Machine à coudre chirurgicale selon la revendication 31, caractérisée en ce que le bras central (35) immobile est fixé au moyen d'un raccord instantané (176) au panneau avant (31) du bâti de la machine.

33. Machine à coudre chirurgicale selon la revendication 31 ou 32, caractérisée en ce que le bras central (35) immobile comporte à son extrémité libre un ergot (38) en forme de plaque pour l'appui des bords de tissu (A, B) adjacents, cet ergot étant muni d'une ouverture de passage (39) pour l'aiguille (1).

34. Machine à coudre chirurgicale selon l'une des revendications précédentes, caractérisée par une pince d'avance (120) qui fonctionne à la même cadence que l'ouverture et la fermeture de la pincette (32) et qui comporte deux branches (121, 122), lesquelles branches
a) peuvent être mises en position fermée pour saisir les bords de tissu (A, B) au voisinage immédiat de la pincette (32) et en position ouverte pour libérer les bords de tissu (A, B) et
b) peuvent être déplacées, alors qu'elles sont en position fermée, pendant chaque période d'ouverture de la pincette (32), hors d'une position de départ dans la direction de l'avance (29) et peuvent être ramenées, alors qu'elles sont en position ouverte, pendant chaque période de fermeture de la pincette (32), dans la position de départ en allant à l'opposé de la direction de l'avance.

35. Machine à coudre chirurgicale selon la revendication 34, caractérisée en ce que les deux branches (121, 122) de la pince sont contraintes en position fermée par au moins un ressort (123) et peuvent être mises en position ouverte contre l'action du ressort par au moins une came radiale (124).

36. Machine à coudre chirurgicale selon la revendication 35, caractérisée en ce que les deux branches (121, 122) de la pince sont contraintes en position fermée par un ressort (123) commun.

37. Machine à coudre chirurgicale selon la revendication 36, caractérisée en ce que la précontrainte du ressort (123) est réglable.

38. Machine à coudre chirurgicale selon la revendication 36 ou 37, caractérisée en ce que le ressort (123) est construit comme un ressort hélicoïdal de compression.

39. Machine à coudre chirurgicale selon l'une des revendications 35 à 38, caractérisée en ce que les deux branches (121, 122) de la pince sont couplées ensemble par un mécanisme de transmission (125) et peuvent être mises en position ouverte par une came radiale (124).

40. Machine à coudre chirurgicale selon la revendication 39, caractérisée en ce que la came radiale (124) est prévue sur un, c'est-à-dire le, cylindre de commande (8) destiné à l'entraînement de l'aiguille (1) et de la navette circulaire (2).

41. Machine à coudre chirurgicale selon l'une des revendications 34 à 40, caractérisée en ce que la position fermée (S'ᵣ ou S'ₗ) de chaque branche (121 ou 122) de la pince est réglable.

42. Machine à coudre chirurgicale selon la revendication 41 et en relation avec la revendication 39 ou 40, caractérisée en ce que seul un levier d'actionnement (151) situé du côté du ressort et du mécanisme de transmission est associé à une première branche (121) de la pince tandis qu'un premier levier d'actionnement (152) situé du côté du ressort et du mécanisme de transmission ainsi qu'un second levier d'actionnement (157) situé du côté de la came radiale sont associés à l'autre branche (122) de la pince et coopèrent par l'intermédiaire d'un élément de réglage (162).

43. Machine à coudre chirurgicale selon la revendication 42, les deux branches de la pince étant contraintes en position fermée par un ressort hélicoïdal de compression commun, caractérisée en ce que le second levier d'actionnement (157) est agencé sur le côté du premier levier d'actionnement (152) qui est éloigné du ressort hélicoïdal de compression (123) et l'élément de réglage (162) est construit sous forme de tige de poussée pour l'appui de l'extérieur contre le premier levier d'actionnement (152) et peut être vissé dans le second levier d'actionnement (157).

44. Machine à coudre chirurgicale selon l'une des revendications 34 à 43, caractérisée en ce que les deux branches (121, 122) de la pince peuvent être déplacées par un moteur électrique pas à pas réversible (126) dans la direction de l'avance (29) et à l'opposé de la direction de l'avance.

45. Machine à coudre chirurgicale selon la revendication 44, caractérisée en ce que le nombre de pas qu'effectue le moteur électrique pas à pas (126) aussi bien dans une direction pour le mouvement d'avance des deux branches (121, 122) de la pince que dans l'autre direction pour le mouvement de recul des deux branches (121, 122) de la pince, est réglable.

46. Machine à coudre chirurgicale selon la revendication 44 ou 45, caractérisée en ce qu'une broche (130 ou 131) est associée à chaque branche (121 ou 122) de la pince, laquelle broche peut être déplacée axialement en un mouvement de va-et-vient au moyen d'un anneau (141 ou 142) qui peut être tourné dans un sens ou dans l'autre par le moteur électrique pas à pas (126) et qui peut être vissé par un taraudage (143 ou 144) sur un filetage correspondant (139 ou 140) de la broche (130 ou 131).

47. Machine à coudre chirurgicale selon la revendication 46, caractérisée en ce qu'un engrenage distributeur (129), comportant une roue dentée primaire (136) entraînée par le moteur électrique pas à pas (126) et deux roues dentées secondaires (137, 138), est placé en amont des deux anneaux (141, 142), les roues dentées secondaires étant chacune en prise avec la roue dentée primaire (136) et étant liées respectivement au premier anneau (141) ou à l'autre anneau (142).

48. Machine à coudre chirurgicale selon la revendication 47, caractérisée en ce que chaque anneau (141 ou 142) et la roue dentée secondaire associée (137 ou 138) sont coaxiaux et sont construits d'une seule pièce.

49. Machine à coudre chirurgicale selon la revendication 47 ou 48, caractérisée en ce qu'un engrenage conique (128) est prévu entre le moteur électrique pas à pas (126) et l'engrenage distributeur (129).

50. Machine à coudre chirurgicale selon la revendication 49, caractérisée en ce que l'engrenage conique (128) est constitué de deux roues coniques (132, 133), mutuellement en prise, qui sont liées respectivement à l'arbre secondaire (134) du moteur électrique pas à pas (126) et à l'arbre primaire (135) de l'engrenage distributeur (129).

51. Machine à coudre chirurgicale selon l'une des revendications 46 à 50, caractérisée en ce que les deux broches (130, 131) peuvent être déplacées en un mouvement de va-et-vient, afin de déplacer les deux branches (121, 122) de la pince en position fermée et en position ouverte, au moyen de deux manchons pivotants (145, 146) qui peuvent tourner dans un sens ou dans l'autre, les deux broches (130, 131) pouvant être déplacées axialement respectivement dans un premier manchon pivotant (145) et dans l'autre manchon pivotant (146) avec une liaison fixe en rotation avec ceux-ci.

52. Machine à coudre chirurgicale selon la revendication 51 et en relation avec la revendication 42 ou 43, caractérisée en ce que les deux manchons pivotants (145, 146) sont munis chacun du levier d'actionnement (151 ou 152) associé à la première branche (121) de la pince ou à l'autre branche (122) de la pince et s'étendant globalement en direction radiale du côté du ressort et du mécanisme de transmission.

53. Machine à coudre chirurgicale selon la revendication 52, caractérisée en ce que le mécanisme de transmission (125) couplant ensemble les deux branches (121, 122) de la pince est conformé en tiges avec deux bras (153, 154) qui s'étendent globalement en direction radiale respectivement depuis le premier manchon pivotant (145) et l'autre manchon pivotant (146) et qui s'enchaînent par leurs extrémités libres au moyen d'une broche transversale (155) et d'un creux (156) en forme de trou oblong pour le logement de la broche transversale (155).

54. Machine à coudre chirurgicale selon la revendication 52 ou 53, caractérisée en ce que le second levier d'actionnement (157) d'une branche (122) de la pince comporte deux bras (159, 160), pour coopérer avec le premier levier d'actionnement (152) de la branche (122) de la pince ou pour coopérer avec la came radiale (124), qui s'étendent globalement en direction radiale depuis un moyeu (158) tubulaire qui est agencé sur la même broche (131) qui s'étend aussi à travers le manchon pivotant (146) muni du premier levier d'actionnement (152).

55. Machine à coudre chirurgicale selon l'une des revendications 46 à 54, les deux branches de la pincette étant prévues respectivement sur un arbre extérieur creux et sur un arbre intérieur logé dans le précédent, caractérisée en ce que les deux broches (130, 131) sont agencées parallèlement à l'arbre extérieur (36) et à l'arbre intérieur (37) et les deux branches (121, 122) de la pince s'étendent globalement en direction radiale respectivement depuis une broche (130) et depuis l'autre broche (131).

56. Machine à coudre chirurgicale selon la revendication 55 et en relation avec la revendication 54, un arbre de réglage parallèle à l'arbre extérieur et à l'arbre intérieur étant prévu pour desserrer la pincette de telle sorte qu'il peut tourner au moyen d'un levier à main, pour écarter les deux branches de la pincette, contre l'action de leur contrainte par ressort, caractérisée en ce que l'arbre de réglage (61) est creux, agencé sur la broche (130) parallèle à la broche (131) qui s'étend à travers le manchon pivotant (146) avec le premier levier d'actionnement (152) et à travers le moyeu (158) tubulaire du second levier d'actionnement (157), et muni d'un bras (170) qui s'étend globalement en direction radiale afin de desserrer la pince d'avance, lequel bras est lié par une attache de poussée (171) au second levier d'actionnement (157) afin d'écarter les deux branches (121, 122) de la pince contre l'action de leur contrainte par ressort lors de la rotation de l'arbre de réglage (61) au moyen du lever à main (62).

57. Machine à coudre chirurgicale selon l'une des revendications 46 à 56, caractérisée en ce que les deux branches (121, 122) de la pince sont fixées chacune au moyen d'un raccord instantané (177 ou 178) respectivement sur la broche (130) et sur l'autre broche (131).

58. Machine à coudre chirurgicale selon l'une des revendications 34 à 57, caractérisée en ce que les deux branches (121, 122) de la pince comportent à leurs extrémités libres chacune un ergot (168) en forme de plaque pour l'appui contre le bord de tissu adjacent et les deux ergots (168) ont leurs côtés (169) en regard conçus de telle sorte que tout glissement entre les bords de tissu saisis et les deux ergots (168) est largement exclu lorsque les deux branches (121, 122) de la pince sont en position fermée.
